(19) Europäisches Patentamt
European Patent Office
Office européen des brevets

(11) Publication number: **0 461 797 A1**

(12) **EUROPEAN PATENT APPLICATION**

(21) Application number: **91305039.9**

(22) Date of filing: **04.06.91**

(51) Int. Cl.5: **C07C 251/60**, C07C 323/12, A01N 37/36

(30) Priority: **14.06.90 GB 9013352**

(43) Date of publication of application: **18.12.91 Bulletin 91/51**

(84) Designated Contracting States: **AT BE CH DE DK ES FR GB GR IT LI LU NL SE**

(71) Applicant: **IMPERIAL CHEMICAL INDUSTRIES PLC**
**Imperial Chemical House, Millbank**
**London SW1P 3JF(GB)**

(72) Inventor: **Bansal, Harjinder Singh**
**15 Chisbury Close**
**Bracknell, Berkshire(GB)**
Inventor: **Kay, Ian Trevor**
**Chy-AN-Ryn, Love Lane , Mousehole**
**Penzance, Cornwall TR19 65X(GB)**

(74) Representative: **Downes, John Edward et al**
**Imperial Chemical Industries PLC Legal**
**Department: Patents PO Box 6**
**Bessemer Road Welwyn Garden City**
**Hertfordshire AL7 1HD(GB)**

(54) **Herbicidal oximethers.**

(57) A compound of formula (I):

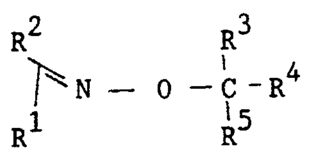

(I)

where $R^1$ is alkyl, optionally substituted aryl or optionally substituted heterocyclyl;
$R^2$ is hydrogen, optionally substituted lower alkyl, lower alkoxy, cycloalkyl, haloalkyl, lower thioalkyl, halo or cyano; or the group $R^1$ is a group of sun formula (i):

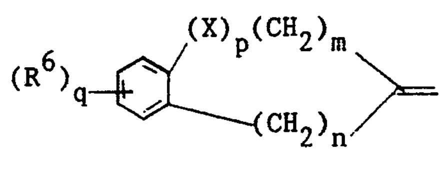

(i)

where q is 0 or an integer of from 1 to 4 and groups $R^6$ are selected independently from hydroxy, alkyl, alkoxy, alkylcarbonyl, halogen, nitrile, nitro, haloalkyl and haloalkoxy; X is oxygen or sulphur, p is 0 or 1, n is 0 or 1 and m is 1, 2 or 3 provided that when n is 0, p + m is 2 or 3 and when n is 1, p + m is 1 or 2;
$R^3$ and $R^4$ are independently selected from hydrogen, halo, alkyl or alkoxy or $R^3$ and $R^4$ together with the carbon atom to which they are attached form a carbocyclic ring; and
$R^5$ is CN; $CO_2R^7$ where $R^7$ is hydrogen, a cation or an esterifying group; a group $-CYNR^8R^9$ where Y is oxygen or sulphur and $R^8$ and $R^9$ are independently selected from hydrogen, hydroxy, alkyl or alkoxy or together with the nitrogen atom to which they are attached form a heterocyclic ring; or $R^8$ together with $R^3$ and the group

EP 0 461 797 A1

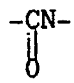

to which it is attached form a heterocyclic ring, or $R^5$ is a group of sub formula (ii)

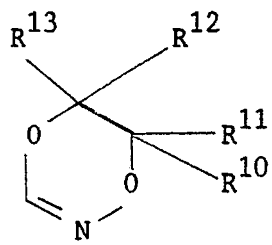

(ii)

where $R^{10}$, $R^{11}$, $R^{12}$ and $R^{13}$ are independently selected from hydrogen or alkyl; or a group S(O)r $R^{14}$ where r is 0, 1 or 2 and $R^{14}$ is hydroxy, lower alkyl or aryl.

The present invention relates to oxime ether derivatives which are useful as herbicides, to process for their preparation and herbicidal compositions containing them.

According to the present invention there is provided a compound of formula (I):
where $R^1$ is alkyl, optionally substituted aryl or optionally substituted heterocyclyl;

$R^2$ is hydrogen, optionally substituted lower alkyl, lower alkoxy, cycloalkyl, haloalkyl, lower thioalkyl, halo or cyano; or the group

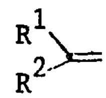

is a group of sub formula (i):
where q is 0 or an integer of from 1 to 4 and groups $R^6$ are selected independently from hydroxy, alkyl, alkoxy, alkylcarbonyl, halogen, nitrile, nitro, haloalkyl and haloalkoxy; X is oxygen or sulphur, p is 0 or 1, n is 0 or 1 and m is 1, 2 or 3 provided that when n is 0, p+ m is 2 or 3 and when n is 1, p+ m is 1 or 2;

$R^3$ and $R^4$ are independently selected from hydrogen, halo, alkyl or alkoxy or $R^3$ and $R^4$ together with the carbon atom to which they are attached form a carbocyclic ring; and

$R^5$ is CN; $CO_2R^7$ where $R^7$ is hydrogen, a cation or an esterifying group; a group $-CYNR^8R^9$ where Y is oxygen or sulphur and $R^8$ and $R^9$ are independently selected from hydrogen, hydroxy, alkyl or alkoxy or together with the nitrogen atom to which they are attached form a heterocyclic ring; or $R^8$ together with $R^3$ and the group -C(O)N- to which it is attached form a heterocyclic ring, or $R^5$ is a group of sub formula (ii) where $R^{10}$, $R^{11}$, $R^{12}$ and $R^{13}$ are independently selected from hydrogen or alkyl; or a group $S(O)_r R^{14}$ where r is 0, 1 or 2 and $R^{14}$ is hydroxy, lower alkyl or aryl.

In particular, the invention provides compounds of formula (I) where $R^1$ is alkyl, optionally substituted aryl or heterocyclyl;

$R^2$ is hydrogen, optionally substituted lower alkyl, lower alkoxy, cycloalkyl, haloalkyl, lower thioalkyl, halo or cyano; or the group

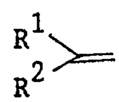

is a group of sub formula (i):
where q is 0 or an integet of from 1 to 4 and groups $R^6$ are selected independently from hydroxy, alkyl, alkoxy, alkylcarbonyl, halogen, nitrile, nitro, haloalkyl and haloalkoxy; X is oxygen or sulphur, p is 0 or 1, n is 0 or 1 and m is 1, 2 or 3 provided that when n is 0, p+ m is 2 or 3 and when n is 1, p+ m is 1 or 2;

$R^3$ and $R^4$ are independently selected from hydrogen, halo, alkyl or alkoxy or $R^3$ and $R^4$ together with the carbon atom to which they are attached form a carbocyclic ring; and

$R^5$ is CN; $CO_2R^7$ where $R^7$ is hydrogen, a cation or an esterifying group; or a group $CONR^8R^9$ where $R^8$ and $R^9$ are independently selected from hydrogen, alkyl or alkoxy or together with the nitrogen atom to which they are attached form a heterocyclic ring; or $R^8$ together with $R^3$ and the group

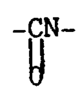

to which it is attached form a heterocyclic ring, or $R^5$ is a group of sub formula (ii):
where $R^{10}$, $R^{11}$, $R^{12}$ and $R^{13}$ are independently selected from hydrogen or alkyl; or a group $S(O)_r R^{14}$ where r is 0, 1 or 2 and $R^{14}$ is hydroxy, lower alkyl or aryl.

As used herein the terms "alkyl" and "alkoxy" refer to straight or branched chain alkyl or alkoxy groups respectively, suitably having from 1 to 10 and preferably from 1 to 6 carbon atoms. The qualification "lower" means that the number of carbon atoms present is suitably from 1 to 4.

Similarly the term "haloalkyl" refers to an alkyl group as defined above substituted by one or more halogen atoms. Suitable halogen atoms for haloalkyl and halo groups include fluorine, chlorine, bromine and iodine.

The term "aryl" used herein includes phenyl and napthyl. The term "heterocyclyl" includes ring

systems having 5 or 6 ring atoms, up to 3 of which are selected from oxygen, nitrogen and sulphur.

Suitably $R^1$ is an optionally substituted aryl in particular phenyl or heterocyclic group.

Examples of substituents for aryl or heterocyclic groups $R^1$ include one or more groups selected from hydroxy, alkyl, alkoxy, alkylcarbonyl, halogen, nitrile, amino, nitro, haloalkyl, haloalkoxy, or a group $S(O)_tR^{15}$ where t is 0, 1 or 2 and $R^{15}$ is alkyl.

Suitable substituents for aryl groups $R^1$ includes one or more groups selected from hydroxy, alkyl, alkoxy, alkylcarbonyl, halogen, nitrile, nitro, haloalkyl and haloalkoxy.

Preferred substituents for aryl groups $R^1$ are $C_{(1-6)}$ alkyl, $C_{(1-6)}$ alkoxy, lower alkyl carbonyl, halo $C_{(1-6)}$ alkoxy or halo wherein the halo groups are selected from fluorine, chlorine, bromine and iodine.

Particular examples of the substituents are methyl, methoxy, ethoxy, bromo, fluoro or chloro.

Further examples include amino or $SO_2CH_3$.

Preferably the substituents are in the ortho or meta position on the phenyl ring, most preferably on the ortho position.

Examples of heterocyclic groups $R^1$ include pyridyl, thiophenyl, pyrrole, furyl and thiazolyl.

Suitable heterocyclic groups $R^1$ include pyridyl, thiophenyl, pyrrole and furyl and thiazolyl.

Suitable substituents for heterocyclic groups $R^1$ include alkyl in particlar methyl. The substituents may be attached to a carbon or heteroatom where possible. In particular the substituted may be attached to a nitrogen atom in the ring.

Suitable optional substituents for alkyl groups $R^2$ include those listed above in relation to aryl or heterocyclic groups $R^1$.

Examples of groups $R^2$ are hydrogen, lower alkyl, lower alkoxy, lower haloalkyl, $C_{3-6}$ cycloalkyl, lower thio alkyl, halo or cyano.

In particular $R^2$ is hydrogen, methyl, ethyl, trifluoromethyl, iso-propyl, methoxy, cyclopropyl or thiomethyl.

Suitably $R^2$ is hydrogen, lower alkyl, lower alkoxy, $c_{3-6}$ cycloalkyl, lower thio alkyl, halo or cyano.

In particular $R^2$ is hydrogen, methyl, ethyl, iso-propyl, methoxy, cyclopropyl, thio methyl or cyano.

When $R^1$ and $R^2$ together form a cyclic group of sub formula (i) suitable groups are those where n is 0. Particular groups are those where n is 0, p is 0 and m is 2 or 3 or n is 0, p is 1 and m is 2.

Preferably X is oxygen.

Suitable groups $R^6$ include those substituents list above for $R^1$. Preferably $R^6$ is halogen such as fluorine. Preferably q is 0 or 1, most preferably 0.

Suitable groups $R^3$ and $R^4$ include hydrogen, lower alkyl, lower alkoxy or halo.

Examples of groups $R^3$ and $R^4$ include halogen, methyl, methoxy, ethyl, ethoxy, iso-propyl or fluoro.

Particular examples of $R^3$ or $R^4$ are hydrogen, methyl, ethyl, methoxy, ethoxy or fluorine.

Preferably $R^3$ and $R^4$ are other than hydrogen.

Where $R^3$ and $R^4$ together form a carbocyclic ring, the ring suitably contains from 3 to 7 carbon atoms.

Suitable groups $R^5$ are $-CYNR^8R^9$ where Y is 0 or S and $R^8$ and $R^9$ are independently selected from hydrogen, lower alkyl such as methyl or ethyl or hydroxy.

Suitable groups $R^5$ are $-CONR^8R^9$ where $R^8$ and $R^9$ are independently selected from lower alkyl such as methyl or ethyl.

When $R^8$ and $R^9$ together with the nitrogen atom to which they are attached form a heterocyclic ring, suitable rings include pyrrolidine, morpholine or azetidine rings.

When $R^8$ together with $R^3$ and the C(O)N group to which it is attached form a heterocyclic ring, it suitably contains from 5 to 7 ring atoms, preferably 5 carbon atoms.

Where $R^5$ is $CO_2R^7$, suitable esterifying groups $R^7$ include alkyl, alkenyl, alkynyl or phenyl any of which may be optionally substituted.

Suitable optional substituents for $R^7$ include one or more groups selected from halo such as fluoro, chloro, bromo or iodo; hydroxy; $C_{1-6}$ alkoxy; nitro; cycloalkyl; heterocyclic optionally substituted by oxo; nitrile; phenyl optionally substituted by nitro, halo such as chloro, alkoxy or carboxy or salts or $C_{1-6}$ alkyl esters thereof; or alkylsilyl groups such as trimethylsilyl.

When $R^7$ is a cation, it is suitably selected from agriculturally acceptable cations such as sodium, potassium, calcium, anomonium or substituted ammonium ions.

Suitable substituted ammonium ions are $\overset{+}{N}R^aR^bR^cR^d$ where $R^a$, $R^b$, $R^c$ and $R^d$ are independently selected from hydrogen or optionally substituted alkyl.

Preferably Y is oxygen.

Preferably $R^5$ is a group $CYNR^8R^9$.

Suitably $R^{10}$, $R^{11}$, $R^{12}$ and $R^{13}$, in sub formula (i) are hydrogen.

When $R^5$ is a group $S(O)_rR^{14}$, $R^{14}$ is suitably lower alkyl in particular methyl or ethyl.

Particular examples of compounds of formula (I) are set out in Tables 1 to 3 below.

TABLE I

| COMPOUND NO. | $R^1$ | $R^2$ | $R^3$ | $R^4$ | $R^5$ |
|---|---|---|---|---|---|
| 1 | $C_6H_5$ | $CH_3$ | H | H | $CON(CH_3)_2$ |
| 2 | $C_6H_5$ | $CH_3$ | $CH_3$ | $CH_3$ | $CON(CH_3)_2$ |
| 3 | $C_6H_5$ | CN | H | $CH_3$ | $CON(CH_3)_2$ |
| 4 | $C_6H_5$ | CH3 | H | $CH_3$ | $CON(CH_2CH_3)_2$ |
| 5 | 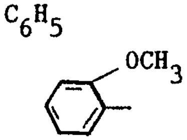 | $CH_3$ | H | $CH_3$ | $CON(CH_3)_2$ |
| 6 | 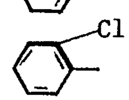 | $CH_3$ | $CH_3$ | $CH_3$ | $CON(CH_3)_2$ |
| 7 | 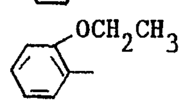 | $CH_3$ | H | $CH_3$ | $CON(CH_3)_2$ |
| 8 | $C_6H_5$ | $CH_2CH_3$ | $CH_3$ | $CH_3$ | $CON(CH_3)_2$ |
| 9 | $C_6H_5$ | $CH_2CH_3$ | H | $CH_3$ | $CON(CH_3)_2$ |
| 10 | 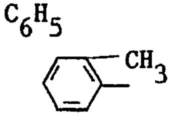 | $CH_3$ | H | $CH_3$ | $CON(CH_3)_2$ |

TABLE I (contd)

| COMPOUND NO. | $R^1$ | $R^2$ | $R^3$ | $R^4$ | $R^5$ |
|---|---|---|---|---|---|
| 11 | 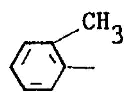 | $CH_3$ | $CH_3$ | $CH_3$ | $CON(CH_3)_2$ |
| 12 | 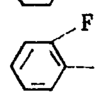 | $CH_3$ | $CH_3$ | $CH_3$ | $CON(CH_3)_2$ |
| 13 | 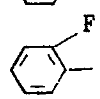 | $CH_3$ | H | $CH_3$ | $CON(CH_3)_2$ |
| 14 | 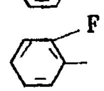 | $CH_3$ | H | $CH_2CH_3$ | $CON(CH_3)_2$ |
| 15 | 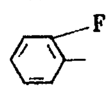 | $CH_3$ | H | H | $CON(CH_3)_2$ |
| 16 | 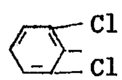 | H | $CH_3$ | $CH_3$ | $CON(CH_3)_2$ |

TABLE I (contd)

| COMPOUND NO. | $R^1$ | $R^2$ | $R^3$ | $R^4$ | $R^5$ |
|---|---|---|---|---|---|
| 17 | 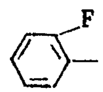 | H | $CH_3$ | $CH_3$ | $CON(CH_3)_2$ |
| 18 | 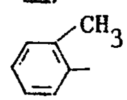 | H | $CH_3$ | $CH_3$ | $CON(CH_3)_2$ |
| 19 | $C_6H_5$ | $CH(CH_3)_2$ | H | $CH_3$ | $CON(CH_3)_2$ |
| 20 | 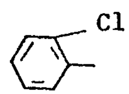 | $CH_3$ | H | H | $CON(CH_3)_2$ |
| 21 | 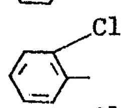 | H | $CH_3$ | $CH_3$ | $CON(CH_3)_2$ |
| 22 | 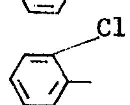 | $CH_3O$ | $CH_3$ | $CH_3$ | $CON(CH_3)_2$ |
| 23 | 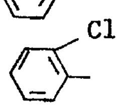 | $CH_3$ | H | $CH_2CH_3$ | $CON(CH_3)_2$ |
| 24 | 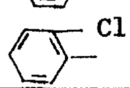 | $CH_3$ | H | $CH_3$ | $CON(CH_3)_2$ |

TABLE I (contd)

| COMPOUND NO. | $R^1$ | $R^2$ | $R^3$ | $R^4$ | $R^5$ |
|---|---|---|---|---|---|
| 25 | 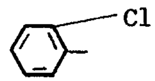 | $CH_3$ | H | $CH_3$ | $CON(CH_2CH_3)_2$ |
| 26 | $C_6H_5$ | $CH_3$ | H | $CH_3$ | $CON(CH_3)_2$ |
| 27 | 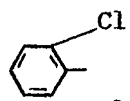 | $CH_3$ | $CH_3$ | $CH_3$ | $CONH_2$ |
| 28 | 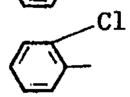 | $CH_3$ | $CH_3$ | $CH_3$ | 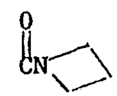 |
| 29 | 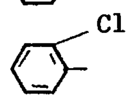 | $CH_3$ | $CH_3$ | $CH_3$ | $CO_2CH_2CH_3$ |
| 30 | 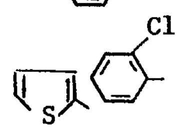 | $CH_3$ | $CH_3$ | $CH_3$ | $CO_2C(CH_3)_2$ |
| 31 |  | $CH_3$ | H | $CH_3$ | $CON(CH_3)_2$ |
| 32 | 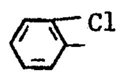 | $CH_3$ | $CH_3$ | $CH_3$ | $CO_2CH_3$ |

TABLE I (contd)

| COMPOUND NO. | $R^1$ | $R^2$ | $R^3$ | $R^4$ | $R^5$ |
|---|---|---|---|---|---|
| 33 | $C_6H_5$ | 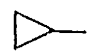 | H | $CH_3$ | $CON(CH_3)_2$ |
| 34 | $C_6H_5$ | 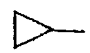 | $CH_3$ | $CH_3$ | $CON(CH_3)_2$ |
| 35 | 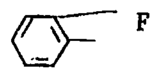 | $CH_3$ | H | $OCH_3$ | $CON(CH_3)_2$ |
| 36 | 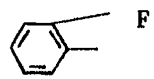 | $CH_3$ | H | $OCH_3$ | $CONH_2$ |
| 37 | 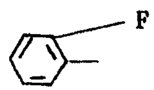 | $CH_3$ | H | $OCH_3$ | CON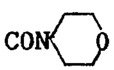 |
| 38 | $C_6H_5$ | $SCH_3$ | H | $CH_3$ | $CON(CH_3)_2$ |
| 39 | $C_6H_5$ | $SCH_3$ | $CH_3$ | $CH_3$ | $CON(CH_3)_2$ |
| 40 | $C_6H_5$ | $CH_3$ | H | F | $CON(CH_3)_2$ |
| 41 | 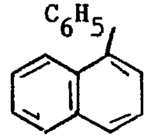 | $CH_3$ | H | $CH_3$ | $CON(CH_3)_2$ |

TABLE I (contd)

| COMPOUND NO. | $R^1$ | $R^2$ | $R^3$ | $R^4$ | $R^5$ |
|---|---|---|---|---|---|
| 42 | 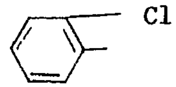 | $CH_3$ | H | $OCH_3$ | $CON(CH_3)_2$ |
| 43 | 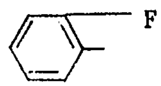 | $CH_3$ | H | $OCH_3$ | $CO_2CH_3$ |
| 44 | 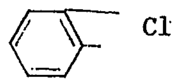 | $CH_3$ | H | $OCH_3$ | $CO_2CH_3$ |
| 45 | $C_6H_5$ | $CH_3$ | H | $CH_3$ | $SO_2CH_2CH_3$ |
| 46 | $C_6H_5$ | $CH_3$ | H | $CH_3$ | $SCH_2CH_3$ |
| 47 | $C_6H_5$ | $CH_3$ | H | $CH_3$ | $SO_2CH_3$ |
| 48 | $C_6H_5$ | $CH_3$ | H | H | $SOCH_3$ |
| 49 | $C_6H_5$ | 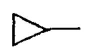 | H | H | $SCH_3$ |
| 50 | $C_6H_5$ | $CH_3$ | H | H | $SCH_3$ |

TABLE I (contd)

| COMPOUND NO. | $R^1$ | $R^2$ | $R^3$ | $R^4$ | $R^5$ |
|---|---|---|---|---|---|
| 51 | $C_6H_5$ | $CH_3$ | H | H | $SO_2CH_3$ |
| 52 | $C_6H_5$ | $CH_2CH_3$ | H | H | $SCH_3$ |
| 53 | Cl 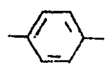 | $CH_2CH_3$ | H | H | $SCH_3$ |
| 54 | $C_6H_5$ | $CH(CH_3)_2$ | H | H | $SCH_3$ |
| 55 | $C_6H_5$ | $(CH_2)_2CH_3$ | H | H | $SCH_3$ |
| 56 | Cl 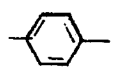 | $CH_3$ | H | H | $SCH_3$ |
| 57 | $CH_3O$ 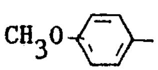 | $CH_2CH_3$ | H | H | $SCH_3$ |
| 58 | $NO_2$ | $CH_3$ | H | H | $SCH_3$ |
| 59 | $C_6H_5$ | H | H | H | $SCH_3$ |

TABLE I (contd)

| COMPOUND NO. | $R^1$ | $R^2$ | $R^3$ | $R^4$ | $R^5$ |
|---|---|---|---|---|---|
| 60 | $C_6H_5$ | $(CH_2)_2CH_3$ | H | H | $S(O)_2CH_3$ |
| 61 | 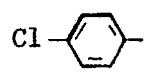 | $CH_3$ | H | H | $S(O)_2CH_3$ |
| 62 | $C_6H_5$ | $CH_3$ | H | $CH_3$ | $SCH_3$ |
| 63 | $C_6H_5$ | $CH_2CH_3$ | H | $CH_3$ | $SCH_3$ |
| 64 | $C_6H_5$ | $CH_2CH_3$ | H | $CH_3$ | $SCH_2CH_3$ |
| 65 | 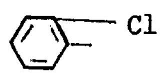 | $CH_3$ | H | H | $SCH_3$ |
| 66 | $C_6H_5$ | $CH_3$ | H | H | $SC_6H_5$ |
| 67 | 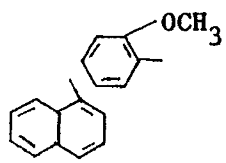 | $CH_3$ | H | H | $SCH_3$ |
| 68 |  | $CH_3$ | H | H | $SCH_3$ |

TABLE I (contd)

| COMPOUND NO. | $R^1$ | $R^2$ | $R^3$ | $R^4$ | $R^5$ |
|---|---|---|---|---|---|
| 69 | 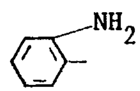 | $CH_3$ | $CH_3$ | $CH_3$ | CON(CH3)2 |
| 70 | $C_6H_5$ | $CF_3$ | $CH_3$ | $CH_3$ | $CON(CH_3)_2$ |
| 71 | 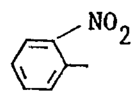 | $CH_3$ | $CH_3$ | $CH_3$ | $CON(CH_3)_2$ |
| 72 | 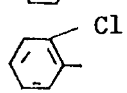 | $CH_3$ | $CH_3$ | $CH_3$ | $CONHCH(CH_3)_2$ |
| 73 | 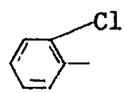 | $CH_3$ | $CH_3$ | $CH_3$ | $CONHC(CH_3)_3$ |
| 74 | 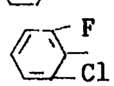 | H | $CH_3$ | $CH_3$ | $CON(CH_3)_2$ |
| 75 | 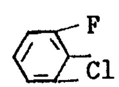 | $CH_3$ | $CH_3$ | $CH_3$ | $CON(CH_3)_2$ |
| 76 | 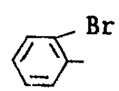 | H | $CH_3$ | $CH_3$ | $CON(CH_3)_2$ |
| 77 | 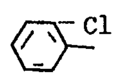 | $CH_3$ | H | $CH(CH_3)_2$ | $CON(CH_3)_2$ |

TABLE I (contd)

| COMPOUND NO. | $R^1$ | $R^2$ | $R^3$ | $R^4$ | $R^5$ |
|---|---|---|---|---|---|
| 78 | 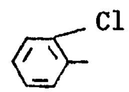 | $CH_3$ | $CH_3$ | $CH_3$ | $CON(CH_3)(C_2H_5)$ |
| 79 | 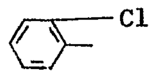 | $CH_3$ | $CH_3$ | $CH_3$ | 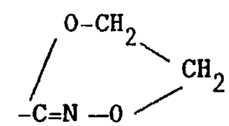 |
| 80 | 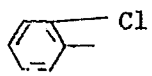 | $CH_3$ | $CH_3$ | $CH_3$ | CONHOH |
| 81 | 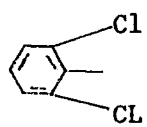 | $CH_3$ | $CH_3$ | $CH_3$ | $CON(CH_3)_2$ |
| 82 | 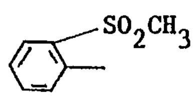 | $CH_3$ | $CH_3$ | $CH_3$ | $CON(CH_3)_2$ |
| 83 | 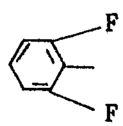 | $CH_3$ | $CH_3$ | $CH_3$ | $CON(CH_3)_2$ |

TABLE I (contd)

| COMPOUND NO. | $R^1$ | $R^2$ | $R^3$ | $R^4$ | $R^5$ |
|---|---|---|---|---|---|
| 84 | 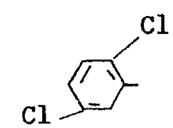 | $CH_3$ | $CH_3$ | $CH_3$ | $CON(CH_3)_2$ |
| 85 | 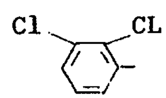 | $CH_3$ | $CH_3$ | $CH_3$ | $CON(CH_3)_2$ |
| 86 | 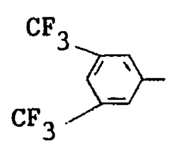 | $CH_3$ | $CH_3$ | $CH_3$ | $CON(CH_3)_2$ |
| 87 | 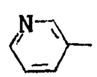 | $CH_3$ | $CH_3$ | $CH_3$ | $CON(CH_3)_2$ |
| 88 | 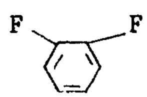 | $CH_3$ | $CH_3$ | $CH_3$ | $CON(CH_3)_2$ |
| 89 | 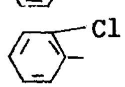 | $CH_3$ | $CH_3$ | $CH_3$ | $CSN(CH_3)_2$ |

TABLE I (contd)

| COMPOUND NO. | $R^1$ | $R^2$ | $R^3$ | $R^4$ | $R^5$ |
|---|---|---|---|---|---|
| 90 | 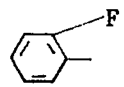 | $CH_3$ | $CH_3$ | $CH_3$ | $CSN(CH_3)_2$ |
| 91 | 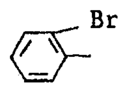 | $CH_3$ | $CH_3$ | $CH_3$ | $CSN(CH_3)_2$ |
| 92 | 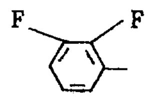 | $CH_3$ | $CH_3$ | $CH_3$ | $CO_2CH_3$ |
| 93 | 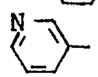 | $CH_3$ | $CH_3$ | $CH_3$ | $CO_2CH_3$ |
| 94 | 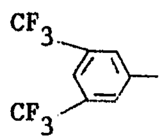 | $CH_3$ | $CH_3$ | $CH_3$ | $CO_2CH_3$ |
| 95 | 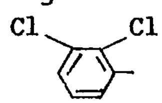 | $CH_3$ | $CH_3$ | $CH_3$ | $CO_2CH_3$ |
| 96 | 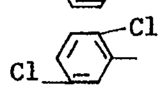 | $CH_3$ | $CH_3$ | $CH_3$ | $CO_2CH_3$ |
| 97 | 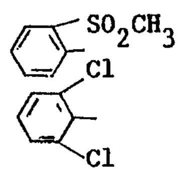 | $CH_3$ | $CH_3$ | $CH_3$ | $CO_2CH_3$ |
| 98 | | $CH_3$ | $CH_3$ | $CH_3$ | $CO_2CH_3$ |

TABLE I (contd)

| COMPOUND NO. | $R^1$ | $R^2$ | $R^3$ | $R^4$ | $R^5$ |
|---|---|---|---|---|---|
| 99 | 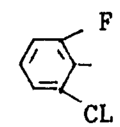 | $CH_3$ | $CH_3$ | $CH_3$ | $CO_2CH_3$ |
| 100 | 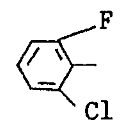 | H | $CH_3$ | $CH_3$ | $CO_2H$ |
| 101 | 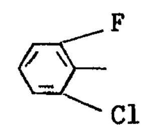 | H | $CH_3$ | $CH_3$ | $CO_2CH_3$ |
| 102 | 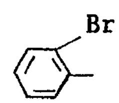 | H | $CH_3$ | $CH_3$ | $CO_2H$ |
| 103 | 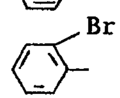 | H | $CH_3$ | $CH_3$ | $CO_2CH_3$ |
| 104 | 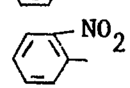 | $CH_3$ | $CH_3$ | $CH_3$ | $CO_2CH_3$ |

TABLE I (contd)

| COMPOUND NO. | $R^1$ | $R^2$ | $R^3$ | $R^4$ | $R^5$ |
|---|---|---|---|---|---|
| 105 | $C_6H_5$ | $CF_3$ | $CH_3$ | $CH_3$ | $CO_2CH_3$ |
| 106 | 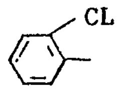 | $CH_3$ | H | $CH_3$ | $CO_2CH_3$ |
| 107 | 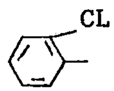 | $CH_3$ | H | $OCH_3$ | $CO_2CH_3$ |
| 108 | 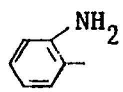 | $CH_3$ | $CH_3$ | $CH_3$ | $CO_2CH_3$ |
| 109 | 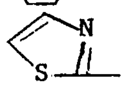 | $CH_3$ | $CH_3$ | $CH_3$ | $CON(CH_3)_2$ |
| 110 | 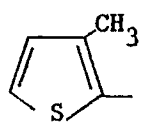 | $CH_3$ | $CH_3$ | $CH_3$ | $CON(CH_3)_2$ |
| 111 | 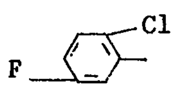 | $CH_3$ | $CH_3$ | $CH_3$ | $CON(CH_3)_2$ |

TABLE I (contd)

| COMPOUND NO. | $R^1$ | $R^2$ | $R^3$ | $R^4$ | $R^5$ |
|---|---|---|---|---|---|
| 112 | 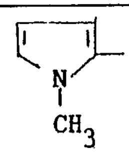 | $CH_3$ | $CH_3$ | $CH_3$ | $CON(CH_3)_2$ |
| 113 | 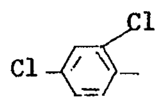 | $CH_3$ | $CH_3$ | $CH_3$ | $CON(CH_3)_2$ |
| 114 | 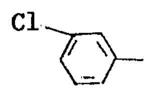 | $CH_3$ | $CH_3$ | $CH_3$ | $CON(CH_3)_2$ |
| 115 | 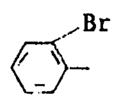 | $CH_3$ | $CH_3$ | $CH_3$ | $CON(CH_3)_2$ |
| 116 | 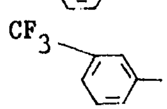 | $CH_3$ | $CH_3$ | $CH_3$ | $CON(CH_3)_2$ |
| 117 | 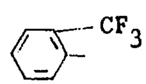 | $CH_3$ | $CH_3$ | $CH_3$ | $CON(CH_3)_2$ |

TABLE I (contd)

| COMPOUND NO. | $R^1$ | $R^2$ | $R^3$ | $R^4$ | $R^5$ |
|---|---|---|---|---|---|
| 118 | 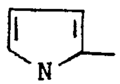 | $CH_3$ | $CH_3$ | $CH_3$ | $CON(CH_3)_2$ |
| 119 | 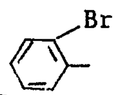 | $CH_3$ | H | $OCH_2CH_3$ | $CON(CH_3)_2$ |
| 120 | 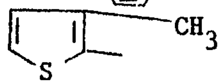 | $CH_3$ | $CH_3$ | $CH_3$ | $CO_2CH_3$ |
| 121 | 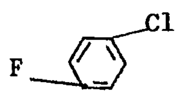 | $CH_3$ | $CH_3$ | $CH_3$ | $CO_2CH_3$ |
| 122 | 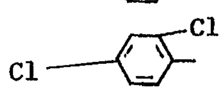 | $CH_3$ | $CH_3$ | $CH_3$ | $CO_2CH_3$ |
| 123 | 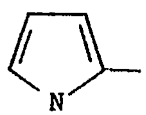 | $CH_3$ | $CH_3$ | $CH_3$ | $CO_2CH_3$ |
| 124 | 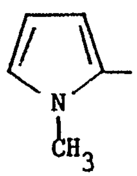 | $CH_3$ | $CH_3$ | $CH_3$ | $CO_2CH_3$ |

TABLE I (contd)

| COMPOUND NO. | $R^1$ | $R^2$ | $R^3$ | $R^4$ | $R^5$ |
|---|---|---|---|---|---|
| 125 | 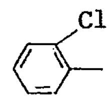 | H | $CH_3$ | $CH_3$ | $CO_2CH_3$ |
| 126 | 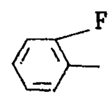 | $CH_3$ | H | $OCH_3$ | $CO_2CH_3$ |
| 127 | 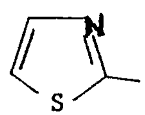 | $CH_3$ | $CH_3$ | $CH_3$ | $CO_2CH_3$ |
| 128 | 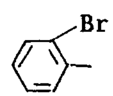 | $CH_3$ | H | $OCH_2CH_3$ | $CO_2CH_2CH_3$ |
| 129 | 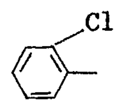 | $CH_3$ | $CH_3$ | $CH_2CH_3$ | $CO_2CH_3$ |
| 130 | 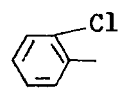 | $CH_3$ | $CH_3$ | $CH_2CH_3$ | $CON(CH_3)_2$ |

TABLE I (contd)

| COMPOUND NO. | $R^1$ | $R^2$ | $R^3$ | $R^4$ | $R^5$ |
|---|---|---|---|---|---|
| 131 | 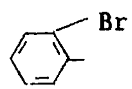 | $CH_3$ | H | $OCH_3$ | $CON(CH_3)_2$ |
| 132 | 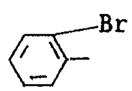 | $CH_3$ | H | $OCH_3$ | $CSN(CH_3)_2$ |
| 133 | $C_6H_5$ | $CH_2OCH_3$ | $CH_3$ | $CH_3$ | $CON(CH_3)_2$ |

TABLE II

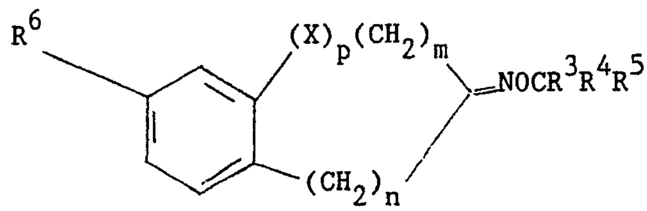

| COMPOUND | m | p | n | X | $R^3$ | $R^4$ | $R^5$ | $R^6$ |
|---|---|---|---|---|---|---|---|---|
| 140 | 3 | 0 | 0 | - | H | $CH_3$ | $CON(CH_3)_2$ | H |
| 141 | 2 | 0 | 0 | - | $CH_3$ | $CH_3$ | $CON(CH_3)_2$ | H |
| 142 | 2 | 0 | 0 | - | H | $CH_3$ | $CON(CH_3)_2$ | H |
| 143 | 2 | 1 | 0 | 0 | H | $CH_3$ | $CON(CH_3)_2$ | H |
| 144 | 2 | 0 | 0 | - | $CH_3$ | $CH_3$ | $CO_2CH_3$ | F |

TABLE III

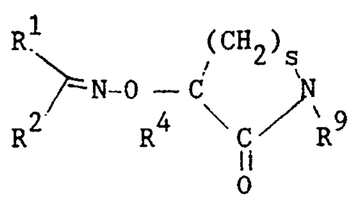

| COMPOUND NO | $R^1$ | $R^2$ | $R^4$ | $R^9$ | s |
|---|---|---|---|---|---|
| 145 | Cl | $CH_3$ | H | $CH_3$ | 2 |

Compounds of formula (I) can be prepared by reacting a compound of formula (II) wherein $R^1$ and $R^2$ are as defined in relation to formula (I); with a compound of formula (III) wherein $R^3$, $R^4$ and $R^5$ are as defined in relation to formula (I) and Z is a leaving group in the presence of a

base.

Suitable bases include strong bases such as alkali metal hydrides in particular sodium hydride. Other suitable bases are weaker bases such as alkali metal carbonates for example potassium carbonate.

The reaction is suitably effected in organic solvent such as dimethylformamide (DMF), dimethylsuphoxide, elevated temperatures for examples of from 20 to 120 °C are suitably employed in the process.

Suitable leaving groups Z include halogen in particular chlorine and bromine, mesylate and tosylate.

Compounds of formula (II) can be prepared by reacting a compound of formula (IV); wherein $R^1$ and $R^2$ are as defined in relation to formula (I) with hydroxylamine or a salt thereof in the presence of a base such as potassium carbonate. The reaction is suitably effected in an organic solvent such as a lower alcohol in particular methanol at temperatures of from 20 to 60 °C.

Compounds of formula (II) and (IV) are known compounds and can be prepared from known compounds by conventional methods.

Compounds of formula (I) where $R^5$ is a group $CO_2R^7$ can be converted to compounds of formula (I) where $R^5$ is $CONR^8R^9$ by (a) removing the group $R^7$, (b) converting the resultant acid to the acid chloride and (c) reacting the acid chloride with an appropriate amine. Each of the steps (a) to (c) involve standard chemical manipulations and suitable conditions and reactions are well known in the art. Processes of this type are included in the examples given hereinafter. Alternatively compounds of formula (I) where $R^5$ is $CO_2R^7$ can be converted to a group where $R^7$ is a different esterifying group by reacting the acid obtained in step (a) above with an appropriate esterifying reagent such as an alcohol in the presence of an acid. Again suitable conditions are exemplified hereinafter.

When $R^5$ is a group $CSNR^8R^9$ it may be prepared by thiolation of the corresponding compound where $R^5$ is $CONR^8R^9$ as exemplified hereinafter.

Compound of formula (I) where $R^8$ is $S(O)_rR^{14}$ and r is 1 or 2 can be prepared by oxidation of corresponding compounds where r is 0. Oxidation is suitably effected by means of conventional oxidising agent such as potassium permanganate in a medium of glacial acetic acid under standard conditions.

Compounds of formula (I) wherein $R^5$ is $CO_2R^7$ can be converted directly to compounds of formula (I) where $R^5$ is $CONR^8R^9$ by reaction with a compound of formula (V)
wherein $R^8$ and $R^9$ are as defined in relation to formula (I) and $R^{16}$ and $R^{17}$ are independently alkyl groups, for example methyl. The reaction is suitably carried out in an inert organic solvent such as dichloromethane at temperatures of from 25 to 50 °C. Suitably the reaction is effected under an inert atmosphere for example of nitrogen.

Compounds of formula (V) can be prepared in situ by reacting a compound of formula (VI)
wherein $R^{16}$ $R^{17}$ and $R^{18}$ are independently alkyl groups with an amine of formula (VII)
wherein $R^8$ and $R^9$ are as defined in relation to formula (I) at low temperatures for example of from 0 to 10 °C

A suitable compound of formula (VI) is trimethylaluminium.

This type of reaction is illustrated in Tet. Letts. 4171, 1977, S Weinreb, Colorado State University. In some cases, the compound of formula (I) where $R^5$ is $CO_2R^7$ can be converted to compounds where $R^5$ is $CONR^8R^9$ by reaction directly with a compound of formula VII. This reaction is particularly useful when $R^8$ and $R^9$ together with the nitrogen atoms to which they are attached form a heterocyclic ring. The reaction is suitably carried out in an inert organic solvent such as methanol at moderate temperature of from 20 to 60 °C, conveniently at ambient temperature.

The compounds of the invention are capable of controlling the growth of a wide variety of plants and in apriicular some show a useful selectivity in crops such as soya, maize, rice and winter wheat. They may be applied to the soil before the emergence of plants (pre-emergence application) or they may be applied to the above ground parts of growing plants (post-emergence application). In general the compounds are more active by pre emergence application. In another aspecy, therefore, the invention provides a process of inhibiting the growth of unwanted plants, by applying to the plants, or to the locus thereof, a compound of the formula (I) as hereinbefore defined. The rate of application required to inhibit the growth of unwanted plants will depend on, for example, the particular compound of formula (I) chosen for use, and the particular species of plant it is desired to control. However, as a general guide, an amount of from 0.01 to 5.0 kilograms per hactare, and preperably 0.025 to 2 kilograms per hectare is usually suitable.

The compounds of the invention are preferably applied in the form of a composition, in which the active ingredient is mixed with a carrier comprising a solid or liquid diluent. In another aspect, therefore, the invention provides a herbicidal composition, comprising as an active ingredient a compound of the formula (I) as hereinbefore defined, in admixture with a solid or liquid diluent. Preferably the composition also comprises a surface active agent.

The solid compositions of the invention may be for example, in the form of dusting powders, or may

take the form of granules. Suitable solid diluents include, for example, kaolin, bentonite, kieselguhr, dolomite, calcium carbonate, talc, powdered magnesia and Fuller's earth. Solid compositions also include soluble powders and granules which may comprise a compound of the invention in admixture with a water soluble carrier.

Solid compositions may also be in the form of dispersible powders or grains comprising in addition to the active ingredient, a wetting agent to facilitate the dispersion of the powder or grains in liquids. Such powders or grains may include fillers, suspending agents and the like.

Liquid compositions include solutions, dispersions and emulsions containing the active ingredient preferably in the presence of one or more surface active agents. Water or organic liquids may be used to prepare solutions, dispersions, or emulsions of the active ingredient. The liquid compositions of the invention may also contain one or more corrosion inhibitors for example lauryl isoquinolinium bromide.

Surface active agents may be of the cationic, anionic or non-ionic type. Suitable agents of the cationic type include for example quaternary ammonium compounds, for example cetyltrimethylammonium bromide. Suitable agents of the anionic type include for example soaps, salts of aliphatic mono-esters of sulphuric acid, for example sodium lauryl sulphate; and salts of sulphonated aromatic compounds, for example dodecylbenzenesulphonate, sodium, calcium and ammonium lignosulphonate, butylnaphthalene sulphonate, and a mixture of the sodium salts of diisopropyl- and triisopropyl-naphthalenesulphonic acid. Suitable agents of the non-ionic type include, for example, the condensation products of ethylene oxide with fatty alcohols such as oleyl alcohol and cetyl alcohol, or with alkyl phenols such as octyl=phenol, nonylphenol, and octylcresol. Other non-ionic agents are the partial esters derived from long chain fatty acids and hexitol anhydrides, for example sorbitol monolaurate; the condensation products of the said partial esters with ethylene oxide and the lecithins; and silicone surface active agents (water soluble surface active agents having a skeleton which comprises a siloxane chaib e.g. Silwet L77). A suitable mixture in mineral oil is Atplus 411F.

The compositions which are to be used in the form of aqueous solutions, dispersions or eulsions are generally supplied in the form of a concentrate containing a high proportion of the active ingredient, the concentrate being diluted with water before use. These concentrates are usally required to withstand storage for prolonged periods and after such storage to be capable of dilution with water in order to form aqueous preparations which remain homogeneous for a sufficient time to enable them to be applied by conventional spray equipment.

The compositions of the invention may contain, in addition to carriers and surface active agents, various other constituents to increase their usefulness. They may contain, for example, buffering salts to maintain the pH of the compositions within a desired range; antifreeze agents, for example oils and humectants; and sequrstrants, for example citric acid and ethylenediaminetetracetic acid, which help to prevent the formation of insoluble precipitates when the compositions are diluted with hard water. Aqueous dispersions may contain anti-settling agents and anti-caking agents. The compositions may in general contain a dye or pigment to impart a characyeristic colour. Agents for increasing viscosity may be added to reduce the formation of fine droplets during spraying, and thereby reduce spray drift. Other additives useful for particular pruposes will be known to those skilled in the formulation art.

In general concentrates may conveniently contain from 10 - 85% and preferably from 25 to 60% by weight of active ingredient. Dilute preparations ready for use may contain varying amounts of the active ingredient, depending upon the purpose for which they are to be used; however, dilute preparations suitable for many uses contain between 0.01% and 10% and preferably between 0.1% and 1% by weight of the active ingredient.

The compositions of the invention may comprise, in addition to one or more compounds of the invention, one or more compounds not of the invention but which possess biological activity. Accordingly in yet a still further embodiment the invention provides a herbicidal composition comprising a mixture of at least one herbicidal compound of formula (I) as hereinbefore defined with at least one other herbicide.

The other herbicide may be any herbicide not having the formula (I). It will generally be a herbicide having a complementary action in the particular application.

Examples of useful complementary herbicides include:

A. benzo-2,1,3-thiadiazin-4-one-2,2-dioxides such as bentazone;

B. hormone herbicides, particularly the phenoxy alkanoic acids such as MCPA, MCPA-thioethyl, dichlorprop, 2,4,5-T, MCPB, 2,4-D, 2,4-DB, mecoprop, trichlopyr, clopyralid, and their derivatives (eg. salts, esters and amides);

C. 1,3 dimethylpyrazole derivatives such as pyrazoxyfen, pyrazolate and benzofenap;

D. Dinitrophenols and their derivatives (eg. acetates) such as dinoterb, dinoseb and its ester, dinoseb acetate;

E. dinitroaniline herbicides such as dinitramine, trifluralin, ethalflurolin, pendimethalin, oryzalin;

F. arylurea herbicides such as diuron, flumeturon, metoxuron, neburon, isoproturon, chlorotoluron, chloroxuron, linuron, monolinuron, chlorobromuron, daimuron, methabenzthiazuron;

G. phenylcarbamoyloxyphenylcarbamates such as phenmedipham and desmedipham;

H. 2-phenylpyridazin-3-ones such as chloridazon and norflurazon;

I. uracil herbicides such as lenacil, bromacil and terbacil;

J. triazine herbicides such as atrazine, simazine, aziprotryne, cyanazine, prometryn, dimethametryn, simetryne, and terbutryn;

K. phosphorothioate herbicides such as piperophos, bensulide, and butamifos;

L. thiolcarbamate herbicides such as cycloate, vernolate, molinate, thiobencarb, butylate*, EPTC* , triallate, di-allate, esprocarb, tiocarbazil, pyridate, and dimepiperate;

M. 1,2,4-triazin-5-one herbicides such as metamitron and metribuzin;

N. benzoic acid herbicides such as 2,3,6-TBA, dicamba and chloramben;

O. anilide herbicides such as pretilachlor, butachlor, alachlor, propachlor, propanil, metazachlor, metolachlor, acetochlor, and dimethachlor;

P. dihalobenzonitrile herbicides such as dichlobenil, bromoxynil and ioxynil;

Q. haloalkanoic herbicides such as dalapon, TCA and salts thereof;

R. diphenylether herbicides such as lactofen, fluroglycofen or salts or ester thereof, nitrofen, bifenox, aciflurofen and salts and esters thereof, oxyfluorfen, fomesafen, chlornitrofen and chlomethoxyfen;

S. phenoxyphenoxypropionate herbicides such as diclofop and esters thereof such as the methyl ester, fluazifop and esters thereof, haloxyfop and esters thereof, quizalofop and esters thereof and fenoxaprop and esters thereof such as the ethyl ester;

T. cyclohexanedione herbicides such as alloxydim and salts thereof, sethoxydim, cycloxyidim, tralkoxydim, and clethodim;

U. sulfonyl urea herbicides such as chlorosulfuron, sulfometuron, metsulfuron and esters thereof; benzsulfuron and esters thereof such as DPX-M6313, chlorimuron and esters such as the ethyl ester thereof pirimisulfuron and esters such as the methyl ester thereof, 2-[3-(4-methoxy-6-methyl-1,3,5-triazinzyl)-3-methylureidosulphonyl) benzoic acid esters such as the methyl ester thereof (DPX-LS300) and pyrazosulfuron;

V. imidazolidinone herbicides such as imazaquin, imazamethabenz, imazapyr and isopropylammonium salts thereof, imazethapyr;

W. arylanilide herbicides such as flamprop and esters thereof, benzoylprop-ethyl, diflufenican;

X. amino acid herbicides such as glyphosate and glufosinate and their salts and esters, sulphosate and bialaphos;

Y. organoarsenical herbicides such as monosodium methanearsonate (MSMA);

Z. herbicidal amide derivative such as napropamide, propyzamide, carbetamide, tebutam, bromobutide, isoxaben, naproanilide and naptalam;

AA. miscellaneous herbicides including ethofumesate, cinmethylin, difenzoquat and salts thereof such as the methyl sulphate salt, clomazone, oxadiazon, bromofenoxim, barban, tridiphane, flurochloridone, quinchlorac and mefanacet;

BB. Examples of useful contact herbicides include:

bipyridylium herbicides such as those in which the active entity is paraquat and those in which the active entity is diquat;

* These compounds are preferably employed in combination with a safener such as dichlormid.

The complementary herbicide is suitably present in the mixture or composition in an amount such that it is applied at its conventional rate.

The following Examples illustrate the invention.

EXAMPLE 1

Preparation of N,N-Dimethyl-2-chloroacetophenoneoxime-0-iso-butyramide (Compound No 6 in Table I)

A solution of 2-chloroacetophenoneoxime (1.695g, 10mmol) in dimethylformamide (DMF) (dry, 4ml) was added dropwise to NaH(50% oil dispersion) (1.1 equiv; 0.264g, 11mmol) suspended in DMF (dry, 15ML) under $N_2$, and the resulting mixture stirred at room temperature for 1 hour. N,N-Dimethyl-$\alpha$-bromoisobutyramide (1.94g, 10mmol) in dry DMF (3ml) was added dropwise to the mixture and the mixture heated at 100°C for 36 hours. Another equivalent of N,N-dimethyl -$\alpha$- bromoisobutyramide (1.94g, 10mmol) in dry DMF (3ml) was added and the resulting mixture heated for a further 20 hours. The reaction

mixture was cooled, poured into $H_2O$ and extracted with EtOAc. The combined organic extracts were washed with brine, dried ($MgSO_4$), filtered and concentrated under reduced pressure to yield an oil residue. The product was purified by flash chromatography (eluted with 50% hexane/$Et_2O$) to afford a white crystalline compound (0.39g, 14%)

$^1$H NMR ($CDCl_3$): 1.6 [6H, s, $C(CH_3)_2$]; 2.25 (3H, s, $CH_3$-C=N); 2.8 [3H, bs, $N(CH_3)_2$], 3.1 [3H, bs, N-$(CH_3)_2$], and 7.3 (4H, m, aromatic C-H).

m.s. M+ 283

EXAMPLE 2

Preparation of Methyl-2-chloroacetophenoneoxime-0-isobutyrate(Compound 32 in Table 1).

2-chloroacetophenoneoxime (4.24g, 25mmol) in DMSO (dry 5ml) was added dropwise to NaH (50% oil dispersion) (1.2 equiv; 0.72g, 30mmol) suspended in DMSO (dry, 15ml) and the resulting mixture stirred at room temperature for 1 hour. Methyl-α-bromoisobutyrate (4.525g, 25mmol) in DMSO (dry, 5ml) was added dropwise to this green solution and the reaction mixture heated at 90°C for 4 hours, then 110°C for a further 8 hours. The mixture was cooled diluted with ice water and extracted with $Et_2O$. The combined organic extracts were washed with brine, dried ($MgSO_4$), filtered and the filtrate concentrated under reduced pressure to yield the product (4.73g, 70%).

$^1$H NMR ($CDCl_3$): 1.6 [6H, s, $C(CH_3)_2$]; 2.25 (3H, s, $CH_3$-C=N); 3.70 (3H, s, $CO_2CH_3$); and 7.3 (4H, m, aromatic C-H).

EXAMPLE 3

Step a

Preparation of 2-chloroacetophenoneoxime-0-isobutyric acid.

Aqueous NaOH (1.2 equiv; 0.48g, 12mmol dissolved in 5ml of $H_2O$) was added dropwise to methyl-2-chloroacetophenoneoxime-0-isobutyrate (2.695g, 10mmol) dissolved in isopropanol (IPA) (25ml) and the resulting solution heated under reflux for 6 hours. The reaction mixture was cooled, IPA removed under reduced pressure, to leave a semi solid residue. $H_2O$ was added and the mixture acidified (2N HCl), and extracted with $Et_2O$. The combined organic extracts were washed with brine, dried ($MgSO_4$), filtered and the filtrate concentrated under reduced pressure to yield the product (2.38g, 93%).

$^1$H NMR ($CDCl_3$): 1.6 [6H, s, $C(CH_3)$]; 2.3 (3H, s, $CH_3$C=N); and 7.3 (4H, m, aromatic C-H).

Step b

Preparation of N,N-Dimethyl-2-chloroacetophenoneoxime-0-isobutyramide (Compound No. 6 in Table 1)

A mixture of 2-chloroacetophenone-0-isobutyric acid (1g, 3.9mmol) and thionylchloride (5ml) was heated at 70°C for 2 hours, and the reaction mixture was concentrated under reduced pressure. The resulting 2-chloroacetophenoneoxime-0-isobutylchloride was dissolved in a mixture of toluene (5ml) and $Et_3N$ (4 drops) and the solution cooled to 0°C (ice bath). Dimethylamine (2 eq., 0.35g, 7.8mmol) in toluene (2ml) was added dropwise and the mixture stirred at room temperature overnight. The reaction mixture was concentrated under reduced pressure, the residue diluted with water and extracted with EtOAC. The combined organic extracts were washed with brine, dried ($MgSO_4$), filtered and the filtrate concentrated under reduced pressure. The product was purified by preparative TLC (eluted with 50% hexane/$Et_2O$) to afford an oil which crystallised on standing (0.85g, 77%).

$^1$H NMR ($CDCl_3$): See Example 1

EXAMPLE 4

Preparation of Azetidine-2-chloroacetophenoneoxime-0-isobutyramide (Compound No. 27 in Table 1).

Trimethylaluminium (2M solution in hexane, 0.27g, 2ml, 3.8mmol) was added dropwise to azetidine (0.23g, 0.3ml, 45mmol) dissolved in $CH_2Cl_2$ (dry, 4ml) at -5°C (ice salt bath) under $N_2$, and the resulting

mixture stirred at room temperature for 30 minutes.
Methyl-2-chloroacetophenoneoxime-0-isobutyrate (1g, 3.7mmol) in $CH_2Cl_2$ (dry, 3ml) was added dropwise and the reaction mixture heated under reflux for 6 hours. The reaction mixture was cooled, concentrated under reduced pressure to yield an oily residue. A mixture of $Et_2O$/EtOAc was added and the solid filtered off, the filtrate was concentrated under reduced pressure, dissolved in warm EtOH, and the insoluble solids again filtered off. The filtrate was concentrated under reduced pressure and the resulting oil triturated with pentane to yield the corresponding product (0.469g, 43%).

$^1$H NMR ($CDCl_3$): 1.5 [6H, s, $C(CH_3)_2$]; 2.2 [5H, m, $CH_3C=N$ and $NCH_2\underline{CH_2}CH_2$]; 4.0 [2H, t, $N\underline{CH_2}CH_2CH_2$]; 4.3 [2H, t, $NCH_2CH_2\underline{CH_2}$];
and 7.3 (4H, m, aromatic C-H)
ms 294 M+.

EXAMPLE 5

Preparation of 2-chloroacetophenoneoxime-0-isobutyronitrile. (Compound No. 28 in Table 1).

1. A mixture of 2-chloroacetophenoneoxime (1.61g, 10mmol),α-bromoisobutyronitrile (1.48g, 10mmol), $K_2CO_3$ (anhydrous, 1.4eq, 2.07g, 15mmol), amd DMSO (dry, 15ml) were heated at 120°C for a period of 28 hours. The reaction mixture was cooled, diluted with $H_2O$ and extracted with $Et_2O$. The combined $Et_2O$ extracts were washed with brine, dried ($MgSO_4$), filtered, and the filtrate concentrated under reduced pressure, to give an oily residue. The product was purified by flash chromatography (eluted with 50% $Et_2O$/hexane) in 14% yield. $^1$H NMR ($CDCl_3$): 1.75 [6H, s, $C(CH_3)_2$], 2.25 (3H, s, $CH_3C=N$; and 7.35 (4H, m, aromatic C-H).
ms 236 M+

2. A mixture of 2-chloroacetophenaneoxime (1.61g, 10mmol), α-bromoisobutyronitrile (1.48g, 10mmol), sodium hydride 10.28g, 12mmol) a catalytic amount of potassium iodide and DMSO (dry, 14ml) were heated at 110°C for a period of 8 hours. The reaction mixture was cooled, diluted with $H_2O$ and extracted with $Et_2O$. The combined $Et_2O$ extracts were washed with brine, dried ($MgSO_4$) filtered, and the filtrate concentrated under reduced pressure, to give an oily residue. After purification by flash chromatography (eluted with 50% $Et_2O$/hexane) the product was obtained (0.41g).

EXAMPLE 6

Preparation of Ethyl-2-chloroacetophenoneoxime-0-isobutyrate. (Compound No. 29 in Table 1).

A mixture of 2-chloroacetophenaneoxime-0-isobutyric acid (0.3g, 12mmol), EtOH (dry, 10ml), and a catalytic amount of p-toluenesulphonic acid were heated under reflux for 2 hours. The reaction mixture was cooled, concentrated under reduced pressure, and the residue dissolved in $Et_2O$. The $Et_2O$ layer was washed with water, dried ($MgSO_4$), filtered and the filtrate concentrated under reduced pressure. The product was purified by preparative TLC (eluted with 50% pentane $Et_2O$) to afford an oil.
$^1$H NMR ($CDCl_3$): 1.25 (3H, t, $OCH_2CH_3$) 1.55 [6H, s, $C(CH_3)_2$]; 2.25 (3H, s, $CH_3$- C=N); 4.20 (2H, q, $OCH_2CH_3$), and 7.30 (4H, m, aromatic C-H).

EXAMPLE 7

Preparation of Iso-propyl-2-chloroacetophenoneoxime-0-isobutyrate. (Compound No. 30 in Table 1).

This compound was prepared as described in Example 6 except that isopropyl alcohol was employed in place of ethanol.
$^1$H NMR ($CDCl_3$): 1.25 [6H, d, $CH(CH)_3)_2$], 1.55 (6H, s, $C(CH_3)_2$], 2.25 (3H, s, $CH_3C=N$], 5.0 (1H, s, $CH(CH_3)_2$], and 7.30 (4H, m, aromatic C-H).

EXAMPLE 8

The following compounds were prepared by methods analogous to those described in Example 1, except that the appropriate α-chloroaceto, propiono or isobutyramide derivatives was employed in place of the N,N-dimethyl-a-bromoisobutyramide:

N,N-Dimethyl-2-fluoroacetophenone-oxime-0-propionamide (Compound No. 13 in Table 1) (oil) structure confirmed by n.m.r.

N,N-Dimethyl-2-fluoroacetophenone-oxime-0-acetamide (Compound No. 12 in Table 1); oil - structure confirmed by n.m.r.

N,N-Dimethyl-2-chloroacetophenone-0-acetamide (Compound No. 20 in Table 1); pale yellow oil; structure confirmed by n.m.r.

N,N-Dimethylacetophenoneoxime-0-acetamide (Compound No. 1 in Table 1); m.p. 65-66 °C.

N,N-Dimethyl-2-chloroacetophenoneoxime-0-propionamide (Compound No. 24 in Table 1); oil, structure confirmed by n.m.r.

N,N-Dimethyl-isobutyrophenoneoxime-0-propionamide (Compound No. 19 in Table 1); oil structure confirmed by n.m.r.

N,N-Dimethyl-α-indaneoneoxime-0-propionomide (Compound No. 142 in Table 2); m.p. 96-97 °C.

N,N-Dimethyl-α-Tetraleneoneoxime-0-propionamide (Compound No. 140 in Table 2).

N,N-Dimethyl-2-Acetylthiopheneoxime-0-propionamide (Compound No. 31 in Table 1); pale yellow oil - structure confirmed by n.m.r.

N,N-Dimethyl-2-methylacetophenoneoxime-0-isobutyamide (Compound No. 11 in Table 1); m.p. 33-88 °C.

N,N-Dimethyl-2-methylacetophenoneoxime-0-propionamide (Compound No. 10 in Table 1); oil; structure confirmed by n.m.r.

N,N-dimethyl-propiophenoneoxime-0-propionamide (Compound No. 9 in Table 1) oil, structure confirmed by n.m.r.

N,N-Dimethyl-2-ethoxyacetophenoneoxime-0-propionamide (Compound No. 7 in Table 1) pale yellow oil, structure confirmed by n.m.r.

N,N-Dimethyl-2-chloroacetophenoneoxime-0-propionamide (Compound No. 25 in Table 1) oil, structure confirmed by n.m.r.

N,N-Diethylacetophenoneoxime-0-propionamide (Compound No. 4 in Table 1) pale yellow oil; structure confirmed by n.m.r.

N,N-Dimethyl-2-methoxyacetophenoneoxime-0-propionamide (Compound No. 5 in Table 1) oil; structure confirmed by n.m.r.

N,N-Dimethyl-4-Chloropropiophenoneoxime-0-propionamide (Compound No. 53 in Table 1) m.p. 70-71 °C.

N,N-Dimethyl-1-acetonaphthoneoxime-0-propionamide (Compound No. 41 in Table 1) pale yellow viscous oil, structure confirmed by n.m.r.

Compound No. 33 in Table 1;
oil, structure confirmed by n.m.r.

Compound No. 44 in Table 1; pale orange oil, structure confirmed by i.r.

## EXAMPLE 9

The following compounds were prepared using methods analogous to those described in Example 1:

N,N-Dimethyl-2-fluoroacetophenoneoxime-0-isobutyramide (Compound No. 12 in Table 1) oil structure confirmed by n.m.r.

N,N-Dimethyl-2-fluorobenzaldoxime-0-isobutyramide (Compound No. 17 in Table 1) oil - structure confirmed by n.m.r.

N,N-Dimethylacetophenoneoxime-0-isobutyramide (Compound No. 2 in Table 1 oil structure confirmed by n.m.r.

N,N-Dimethylpropiophenononeoxime-0-isobutyramide (Compound No. 8 in Table 1) m.p. 49-50 °C.

Compound No. 18 in Table 1; oil, structure confirmed by n.m.r.

Compound No. 21 in Table 1; oil, structure confirmed by n.m.r.

Compound No. 16 in Table 1;
m.pt 108 - 110 °C

Compound No. 23 in Table 1; oil, structure confirmed by n.m.r.

methyl-2-fluoroaceteophenoneoxime-0-methoxyacetate (Compound No. 43 in Table 1).

## EXAMPLE 10

This example illustrates the preparation of N-pyrrolidine-2-fluoroacetophenone oxime-0- methoxyacetamide (Compound No. 36 in Table 1).

Compound 43 (1.4g) prepared by methods analogous to those described in Example 1 was dissolved in methanol (20ml) and pyrrolidine (1.17g) added. The reaction mixture was allowed to stand at ambient

temperature for 18 hours after which the methanol was removed under reduced pressure. The residue was dissolved in hexane, washed with dilute hydrochloric acid, dried using magnesium sulphate and the solvent evaporated under reduced pressure. The product was purified by column chromatography on a silicon column eluted with dichloromethane. The product was then extracted into ethyl acetate which was then removed under reduced pressure to yield the desired product as a colourless oil (yield 0.83g). The structure was confirmed by n.m.r.

EXAMPLE 11

The following compounds were prepared by methods analogues to those described in Example 10:
N-morpholine-2-fluoroacetophenoneoxime-0-methoxy acetoamide (Compound No. 37 in Table 1) oil structure confirmed by n.m.r.
N,N-Dimethyl-2-fluoroacetophenoneoxime-0-methoxyacetolamide (Compound No. 35 in Table I) yellow oil, structure confirmed by n.m.r.
Compound No. 42 in Table 1; oil, structure confirmed by n.m.r.

EXAMPLE 12

This example illustrates the preparation of Compound No. 34 in Table 1

Step a

Hydroxylamine hydrochloride (7.0g), cyclopropyl benzaldehyde (7.0g), dry pyridine (10ml) and ethanol (50ml) was heated together under reflux for 2 hours. The solvent was removed under reduced pressure and water added. The resultant solid was filtered off, dried under suction and recrystallised from carbon tetrachloride/60-80 petroleum ether.

Step b

The product from step (a) (1.89g) was dissolved in dry acetonitrile (30ml) and N,N-dimethyl-α-bromoisobutyramide (2.0g) added together with a catalytic amount of potassium iodide. The mixture was heated under reflux with stirring for 16 hours, then cooled, water added and the mixture extracted into diethyl ether. The ether layer was washed with 5% sodium hydroxide solution (3x15ml), dried over magnesium sulphate and evaporated under reduced pressure. The product was obtained as an oil which crystallised on standing.

After recrystallisation from hexane the desired product was obtained as a white powder (0.43g) m.p. 53-54 °C
Structure confirmed by n.m.r.

EXAMPLE 13

This example illustrates the preparation of Compound 46 in Table 1.

Step (a)

Preparation of (1-chloroethyl)ethylsulphide.

To a stirred solution of diethylsulphide (31.7g) in carbon tetrachloride (100ml) was added chlorine gas (25.0g) in carbon tetrachloride (300ml) dropwise over a period of 45 minutes. A temperature of -20 °C was maintained throughout the addition. After the addition was complete, the reaction was allowed to come to room temperature and then maintained at room temperature for 2 hours during which time hydrogen chloride gas evolved. The solvent was then removed under reduced pressure during which time the temperature was not allowed to exceed 30 °C. The resultant colourless oil was removed and distilled at a 40mm (51 °C).

Step (b)

Acetophenoneoxime (1g), the product from step (a) (0.92g), sodium hydride (0,18g) and dry dimethylsulphoxide (5ml) were stirred together at room temperature for 2 hours. The reaction mixture was then poured into water and extracted into hexane. The hexane layer was washed with 5% sodium hydroxide solution, dried over magnesium sulphate and evaporated under reduced pressure to yield a pale yellow oil. This was distilled on a high vacum pump to give the desired product as a colourless oil (0.5g) which distiiled at 87° C/0.1nm. The structure was confined by n.m.r.

EXAMPLE 14

The following compounds were prepared by methods andogous to that described in Example 13.
Compound 55 in Table 1; structure confined by n.m.r.

EXAMPLE 15

Preparation of Compound No. 60 in Table I.
Compound 55 (2.0g) in glacial acetic acid (20ml) cooled in a water bath and potassium permanganate (1.84g) in water (50ml) dripped in over a period of 15 minutes. The reaction mixture was stirred at room temperature for 2 hours after which sulphur dioxide gas was passed through the solution. Water (25ml) was added and the reaction mixture extracted into trichloromethane (50ml) dried over magnesium sulphate and the solvent removed under reduced pressure to give the desired product as a colourless oil. (Structure confined by n.m.r.).

EXAMPLE 16

The following compounds were prepared by methods analogous to these described in Example 15:
Compound 47 in Table 1; m.pt. 63-65° C
Compound 45 in Table 1; m.pt. 50-51° C

EXAMPLE 17

The following compounds were prepared by a method analogous to that described in Example 4 except that dimethylamine was used instead of azelidine:
N,N-Dimethyl-2-aminoacetophenone oxime-0-isobutyramide (Compound No. 69 in Table 1) - Pale brown solid - structure confined by n.m.r.
N,N-Dimethyl-trifluoroacetophenone-0-isobutyramide (Compound No. 70 in Table 1) oil - structure confirmed by n.m.r.
N,N-Dimethyl-2-nitroacetophenone oxime-0-isobutyramide (Compound No. 71 in Table 1) - oil - structure confirmed by nmr.
N,N-Dimethyl-2-chloro,6-trifluoroacetophenone oxime-0-isobutyramide (Compound No. 75 in Table 1) oil - structure confirmed by n.m.r.
Compound No. 77 in Table 1 - oil structure confirmed by n.m.r.
N,N-Dimethyl-2,6-dichloroacetophenone oxime-0-isobutyramide (Coimpound No. 81 in Table 1) - oil structure confirmed by n.m.r.
N,N-Dimethyl-2-methylsulphonacetophenone oxime-0-isobutyramide (Compound No. 82 in Table 1) - off white solid - structure confirmed by n.m.r.
N,N-Dimethyl-2,6-difluoroacetophenone oxime-0-isobutyramide (Compound No. 83 in Table 1) - oil structure confirmed by n.m.r.
N,N-Dimethyl-2,5-dichloroacetophenone oxime-0-isobutyramide (Compound No. 84 in Table 1) - oil structure confirmed by n.m.r.
N,N-Dimethyl-2,3-dichloroacetophenone oxime-0-isobutyramide (Compound No. 85 in Table 1) - oil, semisolid on standing - structure confirmed ny n.m.r.
N,N-Dimethyl-3,5-bis-trifluoromethylacetophenone-oxime-0-isobutramide (Compound No. 86 in Table 1) oil - structure confirmed by n.m.r.
Compound No. 87 in Table 1 - oil-structure confirmed by n.m.r.
N,N-Dimethyl-2,3-difluoroacetophenone oxime-0-isobutyramide (Compound No. 88 in Table 1) - oil, semisolidified on standing - structure confirmed by n.m.r. and m.s.

EXAMPLE 18

The following compounds were prepared substantially as described in Example 4 except that dimethylamine was used instead of azetidine and slightly different reaction conditions were used. In particular the reaction mixture was stirred at room temperature instead of under reflux and work-up was effected by dilution with water, extraction into ethyl acetate and dried over magnesium sulphate.

Compound No. 109 in Table 1 - orange oil structure confirmed by n.m.r.
Compound No. 110 in Table 1 - pale yellow oil structure confirmed by n.m.r.
Compound No. 111 in Table 1 - orange oil structure confirmed n.m.r.
Compound No. 112 in Table 1 - pale yellow oil structure confirmed by n.m.r.
Compound No. 113 in Table 1 - pale yellow oil structure confirmed by n.m.r.
Compound No. 114 in Table 1 - orange oil - structure confirmed by n.m.r.
Compound No. 115 in Table 1 - orange oil structure confirmed by n.m.r.
Compound No. 116 in Table 1 - orange oil structure confirmed by n.m.r.
Compound No. 117 in Table 1 - orange oil structure confirmed by n.m.r.
Compound No. 118 in Table 1 - dark orange oil structure confirmed by n.m.r.
Compound No. 119 in Table 1 - oil structure confirmed by n.m.r.
Compound No. 130 in Table 1 - orange oil structure confirmed by n.m.r.
Compound No. 131 in Table 1 - oil structure confirmed by n.m.r.
Compound No. 133 in Table 1 - oil structure confirmed by n.m.r.

EXAMPLE 19

The following examples were prepared using the method of Example 3 step (b) except that where necessary dimethylamine was replaced by the appropriate alkylamine:

N-isopropyl-2-chloroacetophenoneoxime-0-isobutyramide (Compound No. 72 in Table 1) - oil - structure confirmed by n.m.r.

N-t-butyl-2-chloroacetophenone oxime-0-isobutyramide (Compound No. 73 in Table 1 - oil stricture confirmed by n.m.r.

Compound No. 74 in Table 1 - oil structure confirmed by n.m.r.
Compound No. 76 in Table 1, - oil structure confirmed by n.m.r.

N-Ethyl-N-methyl-2-chloroacetophenone oxime-0-isobutyramide (Compound No. 78 in Table 1) - oil structure confirmed by n.m.r.

EXAMPLE 20

This example illustrates the preparation of Compound No. 80 in Table 1.

To a stirred mixture of hydroxylamine hydrochloride (2 equiv, 0.695g, 10mmol) in dry methanol ($10cm^3$) was added potassium hydroxide (3 equiv) (0.84g, 15mmol in methanol ($3cm^3$)). This mixture was stirred for ½ hour at room temperature, then a solution of methyl-2-chloroacetophenone oxime-0-isobutyrate (1.35g, 50mmol) in methanol ($10cm^3$) added dropwise.

The whole mixture was stirred over the weekend. A white precipitate was filtered off and the filtrate concentrate to give an oily residue.

The residue was dissolved in water and acidified with dilute HCL. The aqueous mixture was extracted into ethyl acetate and the extract washed, dried and concentrated to give an oil which was purified by preparative TLC. After trituration with pentane a white solid was obtained.

$^1H$ NMR ($CDCl_3$): 1.55[(6H,s,$C(CH_3)_2$], 2.25(3H,s,$\underline{CH_3}$-C=N), 7.35 (4H,m,aromatic C-H), 8.90(1H,s,NH or OH).

EXAMPLE 21

This example illustrates the preparation of Compound No. 79 in Table 1.

Compound No. 80 prepared as described in Example 20, (0.249g, 9.2mmol) was dissolved in dry DMF ($5cm^3$) and 1,2-dibromoethane (0.52g, 27.6mmol) added dropwise, followed by potassium carbonate (0.26g, 18.4mmol). The resulting mixture was stirred overnight. The reaction mixture was diluted with water and extracted with ethyl acetate. The extract was washed, dried and concentrated to give a viscous yellow oil. Trituration with pentane gave the desired compound as a white solid that was filtered off and dried.

$^1H$ NMR ($CDCl_3$): 1.55(6H,s,$C(CH_3)_2$), 2.25(3H,s,($\underline{CH_3}$C=N), 4.20(4H,dt,2x$CH_2$(O$\underline{CH_2}\underline{CH_2}$O), 7.30(4H, m, aromatic $\underline{C-H}$).

EXAMPLE 22

This example illustrates the preparation of N,N-Dimethyl-2-chloroacetophenoneoxime-0-Isobutyrothioamide (Compound No. 89 in Table 1).

To a solution of the N,N,Dimethyl-2-chloroacetophenoneoxime-0-isobutyramide (0.29g, 1.03mmol) in dry toluene ($10cm^3$) was added Lawesson's Reagent (0.416g, 1.03mmol) and the resulting mixture stirred and refluxed for 7 hours. The mixture was then concentrated to give a yellow oil residue which was applied to preparative TLC plates (silica, eluant: $Et_2O$ (1)/Hexane(1)).

The relevant band (top) was isolated to give the desired product as a yellow viscous oil.

$^1H$ NMR ($CDCl_3$): 1.0(6H,s,c($(CH_3)_2$), 2.24(3H,s,$CH_3$-C=N), 3.47(3H,s,$CH_3$ of N($CH_3$)$_2$, 3.56(3H,s,$CH_3$ of N-($CH_3$)$_2$, 7.3(4H,m,aromatic C-H).

EXAMPLE 23

The following compounds were prepared by method analogous to those described in Example 22:

N,N-Dimethyl-2-Fluoroacetophenone oxime-0-isobutyrothioamide. (Compound No. 90 in Table 1) - yellow viscous oil, structure confirmed by n.m.r.

N,N-Dimethyl-2-Bromoacetophenoneoxime-0-isobutyrothioamide. (Compound No. 91 in Table 1) - yellow viscous oil structure confirmed by n.m.r.

N,N-Dimethyl-2-Bromoacetophenone oxime-0-methoxacetamide (Compound No. 132 in Table 1) - oil structure confirmed by n.m.r.

EXAMPLE 24

The following Example was prepared by methods analoguos to those set out in Example 2 modified as necessary. In all cases the structure was confirmed by n.m.r.

Methyl-2-3-difluoroacetophenoneoxime-0-isobutyrate (Compound No. 92 in Table 1) oil.

Compound No. 93 in Table 1 - yellow oil.

Methyl-3,5-bis-trifluoromethylacetophenone oxime-0- isobutyrate (Compound No. 94 in Table 1) oil, that solidified on standing.

Methyl-2,3-dichloroacetophenoneoxime-0-isobutyrate (Compound No. 95 in Table 1). oil, which on standing and trituration with pentane gave a crystalline solid.

Methyl-2,5-dichloroacetophenone oxime-0-isobutyrate (Compound No. 96 in Table 1) oil.

Compound No. 97 in Table 1 - oil

Methyl-2,6-dichloroacetophenone oxime-0-isobutyrate (Compound No. 98 in Table 1) - oil

Methyl-2-chloro-6-fluoroacetophenone oxime-0- isobutyrate (Compound No. 99 in Table 1) oil . (Except that in the method 2-chloro,6-fluoroacetophenone oxime was used instead of the 2-chloroacetophenoneoxime).

Compound No. 101 in Table 1 (except that methyl ethyl ketone and $K_2CO_3$ was used instead of NaH/DMSO).

Compound No. 103 in Table 1 - oil (except that 2-butanone and $K_2CO_3$ was used instead of NaH/DMSO).

Methyl-2-Nitroacetophenone oxime-0-isobutyrate (Compound No. 104 in Table 1) - viscous oil.

[2-Nitroacetoacetophenone oxime was used instead of 2-Chloroacetophenone oxime].

Methyl-trifluoroacetophenone oxime-0-isobutyrate (Compound No. 105 in Table 1) - oil.

Methyl-2-chloroacetophenone oxime-0-propionate (Compound No. 106 in Table 1) - oil. Methyl-2-bromopropionate is as used instead of methyl-α-bromoisobutyrate.

Methyl-2-chloroacetophenone oxime-0-methoxy-acetate. (Compound No. 107 in Table 1) [except THF was used instead of DMSO].

Methyl-2-aminoacetophenone oxime-0- isobutyrate. (Compound No. 108 in Table 1 - oil.

Compound No. 144 in Table II - (equimolar amounts of starting materials and NaH used).

Compound No. 120 in Table 1 - (equimolar amounts of starting materials and NaH used with no heating at all).

Compound No. 121 in Table 1 - (equimolar amounts of starting materials used, with no heating).

Compound No. 122 in Table 1 - (equimolar amounts of starting materials with no heating).

Compound No. 123 in Table 1 - (equimolar amounts of starting materials used with no heating).

Compound No. 124 in Table 1 - (equimolar amounts of starting materials used with no heating).

Compound No. 125 in Table 1 - (equimolar amounts of all reagents without heat).

Compound No. 126 in Table 1 - (equimolar amounts of all reagents heated to 60° C for 6 hours).

Compound No. 127 in Table 1.

Compound No. 128 in Table 1.
Compound No. 129 in Table 1.

EXAMPLE 25

The following compounds were prepared by a method analogous to that described in Example 3 step a. Compound No. 100 in Table 1 - semi-solid. $^1H$ NMR ($CDCl_3$): 1.65(6H,s,$C(CH_3)_2$), 7.15(3H,m,aromatic C-H), 8.45(1H,s,-CH=N-O). Compound No. 102 in Table 1 - semi-solid. $^1H$ NMR ($CDCl_3$): 1.65ppm (6H,s,$c(CH_3)_2$), 7.25-7.70(4H,m,aromatic C-H), 8.55(1H,s,-CH=N-O).

Biological Data

The herbicidal properties of compounds of the invention was tested as described below.

Each compound was formulated for test by mixing an appropriate amount of it with 5ml of an emulsion prepared by diluting 160ml of a solution containing 21.8 grams per litre of Span 80 and 78.2 grams per litre of Tween 20 in methylcyclohexanone to 500ml with water. Span 80 is a Trade Mark for a surface-active agent comprising sorbitan monolaurate. Tween 20 is a Trade Mark for a surface-active agent comprising a condensate of 20 molar proportions of ethylene oxide with sorbitan monolaurate. The mixture of the compound and the emulsion was then shaken with glass beads and diluted to 40ml with water. The spray composition so prepared was sprayed on to young pot plants (post-emergence test) of the species named in Table 2 below, at a rate of equivalent to 1000 litres per hectare. Damage to plants was assessed 14 days after spraying by comparison with untreated plants, on a scale of 0 - 5 where 0 is O to 20% damage and 5 is complete kill. In the table of results a dash (-) means that no test was made.

A test was also carried out to detect pre-emergence herbicidal activity. Seeds of the test species were placed on the surface of fibre trays of soil and were sprayed with the compositions at the rate of 1000 litres per hectate. The seeds were then covered with further soil. Three weeks after spraying, the seedlings in the sprayed fibre trays were compared with the seedlings in unstrayed control trays, the damage being assessed on the same scale of 0 to 5.

The results of the tests are given in Table IV and V below, where Table IV shows the results on the 0-5 scale and Table V shows the results on a percentage scale.

TABLE IV

| COMPOUND NO | RATE OF APPLN kg/ha | PRE- OR POST- EMERGENCE APPLN. | Sb | Rp | Ct | Sy | Mz | Ww | Rc | Sn | Ip | Am | Pi | Ca | Po | Xa | Ab | Cv | Av | Ot | Dg | Pu | Al | St | Ec | Sh | Ag | Cn |
|---|---|---|---|---|---|---|---|---|---|---|---|---|---|---|---|---|---|---|---|---|---|---|---|---|---|---|---|---|
| 1 | 5 | Pre | 2 | 4 | 0 | 3 | 3 | 4 | 3 | 4 | 4 | 4 | 4 | 4 | 4 | - | 3 | - | - | 4 | 4 | 5 | - | 5 | 5 | 3 | 4 | 5 |
| | | Post | 0 | 2 | 1 | 2 | 2 | 2 | 0 | 1 | 1 | 1 | 2 | 4 | 2 | 0 | 2 | 0 | 1 | - | 4 | 1 | - | 3 | 3 | 3 | 1 | 2 |
| 2 | 5 | Pre | 3 | 4 | 2 | 4 | 4 | 5 | 4 | 4 | 0 | 4 | 4 | 4 | - | - | 4 | - | - | 5 | 5 | 5 | - | 5 | 5 | 4 | 3 | 5 |
| | | Post | 2 | 3 | 3 | 4 | 2 | 2 | 0 | 3 | 2 | 3 | 2 | 3 | 4 | - | 3 | 0 | 1 | - | - | 3 | - | 3 | 3 | 5 | 2 | 2 |
| 4 | 5 | Pre | 3 | 2 | 0 | 2 | 0 | 1 | 0 | 1 | 0 | 2 | 3 | 3 | 2 | 0 | 0 | - | - | 1 | 4 | 4 | - | 4 | 4 | 0 | 0 | 0 |
| | | Post | 2 | 2 | 0 | 4 | 0 | 0 | 3 | 1 | 0 | - | 1 | 4 | - | - | 2 | 0 | 0 | - | - | 1 | - | 4 | 2 | 0 | 0 | 0 |

TEST PLANTS (see Table VI)

TABLE IV (cont/d)

| COMPOUND NO | RATE OF APPLN kg/ha | PRE- OR POST-EMERGENCE APPLN. | TEST PLANTS (see Table VI) | | | | | | | | | | | | | | | | | | | | | | | | | |
|---|---|---|---|---|---|---|---|---|---|---|---|---|---|---|---|---|---|---|---|---|---|---|---|---|---|---|---|---|
| | | | Sb | Rp | Ct | Sy | Mz | Ww | Rc | Sn | Ip | Am | Pi | Ca | Po | Xa | Ab | Cv | Av | Ot | Dg | Pu | Al | St | Ec | Sh | Ag | Cn | Sb |
| 5 | | Pre | 2 | 4 | 1 | 4 | 4 | 3 | 0 | 4 | 0 | 4 | 4 | 4 | 3 | - | 4 | - | - | 3 | 4 | 4 | - | 5 | 5 | 3 | 5 | 5 | |
| | | Post | 0 | 3 | 2 | 4 | 2 | 0 | 0 | 2 | 1 | 1 | 1 | 4 | 0 | 1 | 3 | 0 | 0 | - | 4 | 0 | - | 2 | 4 | 1 | 0 | 1 | |
| 6 | 1 | Pre | 4 | 4 | 1 | 3 | 4 | 5 | 4 | 4 | 0 | 5 | 4 | 4 | 4 | - | 4 | - | - | 4 | 5 | 5 | - | 5 | 5 | 4 | 5 | 5 | |
| | | Post | 3 | 4 | 2 | 4 | 3 | 3 | 0 | 3 | 2 | 3 | 2 | 3 | 1 | 4 | 2 | 0 | 0 | 4 | 2 | - | 3 | 5 | 3 | 3 | 2 | 3 | |
| 7 | 5 | Pre | 0 | 1 | 0 | 1 | 1 | 0 | 1 | 2 | 0 | 2 | 2 | 1 | 1 | 0 | 0 | - | - | 1 | 4 | 4 | - | 1 | 3 | 1 | 0 | 0 | |
| | | Post | 2 | 1 | 0 | 4 | 0 | 0 | 0 | 0 | 0 | - | 1 | 4 | - | - | 1 | 0 | 0 | - | - | 0 | - | 1 | 1 | 0 | 0 | 0 | |
| 8 | 3 | Pre | 3 | 3 | 0 | 3 | 0 | 3 | 3 | 4 | 0 | 4 | 4 | 4 | 4 | - | 3 | - | - | 2 | 3 | 5 | - | 4 | 4 | 0 | 2 | 0 | |
| | | Post | 2 | 2 | 0 | 4 | 0 | 0 | 2 | 1 | 1 | - | 3 | 4 | - | - | 2 | 0 | 0 | - | - | 0 | - | 3 | 3 | 3 | 1 | 3 | |

Table IV (cont/d)

| COMPOUND NO | RATE OF APPLN kg/ha | PRE- OR POST-EMERGENCE APPLN. | Sb | Rp | Ct | Sy | Mz | Ww | Rc | Sn | Ip | Am | Pi | Ca | Po | Xa | Ab | Cv | Av | Ot | Dg | Pu | Al | St | Ec | Sh | Ag | Cn |
|---|---|---|---|---|---|---|---|---|---|---|---|---|---|---|---|---|---|---|---|---|---|---|---|---|---|---|---|---|
| 9 | 5 | Pre | 2 | 3 | 0 | 3 | 2 | 3 | 2 | 4 | 0 | 4 | 4 | 4 | 5 | - | 3 | - | - | 3 | 5 | 4 | - | 4 | 4 | 2 | 1 | 0 |
| | | Post | 3 | 3 | 2 | 4 | 0 | 0 | 3 | 3 | 1 | - | 4 | 4 | - | - | - | 3 | 0 | 0 | - | 0 | - | 3 | 3 | 0 | 0 | 3 |
| 10 | 5 | Pre | 4 | 4 | 3 | 4 | 4 | 3 | 2 | 4 | 0 | 4 | - | 4 | 4 | 2 | 4 | - | - | 2 | 4 | 4 | - | 4 | 5 | 3 | 5 | 4 |
| | | Post | 2 | 3 | 3 | 3 | 2 | - | 0 | 4 | 0 | 2 | 3 | 4 | 1 | 2 | 3 | 0 | - | - | 3 | 0 | - | 4 | 4 | - | 1 | 2 |
| 12 | 1 | Pre | 3 | 4 | 0 | 2 | 3 | 3 | 3 | 4 | 0 | 4 | - | 4 | 4 | 1 | 3 | - | - | 5 | 5 | 5 | - | 5 | 5 | 4 | 0 | 0 |
| | | Post | 2 | 1 | 0 | 3 | 3 | 3 | 2 | 5 | 0 | 2 | 3 | 4 | 2 | 4 | 3 | 0 | - | - | 2 | 3 | - | 4 | 4 | - | 0 | 2 |
| 13 | 5 | Pre | 4 | 4 | 2 | 4 | 4 | 4 | 3 | 4 | 0 | 4 | - | 4 | 4 | - | 4 | - | - | 5 | 5 | 5 | - | 5 | 5 | 4 | 5 | 4 |
| | | | 3 | 4 | 2 | 4 | 3 | - | 2 | 4 | 0 | 3 | 3 | 4 | 3 | 1 | 3 | 0 | 0 | - | 4 | 4 | - | 4 | 5 | - | 2 | 2 |

Table IV (cont/d)

| COMPOUND NO | RATE OF APPLN kg/ha | PRE- OR POST-EMERGENCE APPLN. | Sb | Rp | Ct | Sy | Mz | Ww | Rc | Sn | Ip | Am | Pi | Ca | Po | Xa | Ab | Cv | Av | Ot | Dg | Pu | Al | St | Ec | Sh | Ag | Cn |
|---|---|---|---|---|---|---|---|---|---|---|---|---|---|---|---|---|---|---|---|---|---|---|---|---|---|---|---|---|
| 14 | 5 | Pre | 4 | 4 | 2 | 4 | 4 | 5 | 5 | 4 | 0 | 4 | - | 4 | 4 | 3 | 4 | - | - | 5 | 5 | 5 | - | 5 | 5 | 4 | 5 | 5 |
| | | Post | 2 | 4 | 4 | 4 | 3 | 4 | 2 | 4 | 2 | 3 | 3 | 4 | 3 | 2 | 3 | 1 | 4 | - | 3 | 4 | - | 4 | 5 | - | 3 | 2 |
| 15 | 5 | Pre | 3 | 4 | 3 | 4 | 4 | 5 | 5 | 4 | 1 | 4 | 4 | 4 | 4 | 0 | 3 | - | - | 5 | 4 | 5 | - | 5 | 5 | 4 | 5 | 5 |
| | | Post | 2 | 3 | 1 | 3 | 1 | 3 | 2 | 4 | 0 | 2 | 1 | 4 | 0 | 0 | 3 | 0 | 2 | - | 4 | 3 | - | 2 | 3 | 0 | 3 | 3 |
| 16 | 5 | Pre | 5 | 5 | 0 | 2 | 3 | 5 | 4 | 4 | 0 | 5 | 5 | 5 | 4 | 0 | 4 | - | - | 5 | 4 | 5 | - | 5 | 4 | 4 | - | 3 |
| | | Post | 2 | 2 | 0 | 1 | 2 | 0 | 0 | 0 | 0 | 2 | 1 | 4 | 2 | 2 | 4 | 0 | 0 | - | 3 | 0 | - | 5 | 3 | 0 | 0 | 0 |
| 17 | 5 | Pre | 3 | 3 | 0 | 1 | 4 | 5 | 5 | 4 | 0 | 2 | 4 | - | 4 | 2 | 4 | - | - | 4 | 5 | 5 | - | 5 | 4 | 4 | 3 | 2 |
| | | Post | 0 | 3 | 0 | 2 | 3 | - | 1 | 1 | 0 | 1 | 0 | 4 | 1 | 2 | 2 | 0 | 2 | - | 4 | 2 | - | 4 | 3 | 2 | 2 | 2 |

TEST PLANTS (see Table VI)

Table IV (cont/d)

| COMPOUND NO | RATE OF APPLN kg/ha | PRE- OR POST-EMERGENCE APPLN. | TEST PLANTS (see Table VI) | | | | | | | | | | | | | | | | | | | | | | | | | |
|---|---|---|---|---|---|---|---|---|---|---|---|---|---|---|---|---|---|---|---|---|---|---|---|---|---|---|---|---|
| | | | Sb | Rp | Ct | Sy | Mz | Ww | Rc | Sn | Ip | Am | Pi | Ca | Po | Xa | Ab | Cv | Av | Ot | Dg | Pu | Al | St | Ec | Sh | Ag | Cn |
| 18 | 5 | Pre | 3 | 4 | 2 | 3 | 4 | 5 | 5 | 4 | 0 | 5 | 4 | 4 | 4 | 2 | 4 | - | - | 5 | 5 | 5 | - | 5 | 5 | 4 | 3 | 5 |
| | | Post | 1 | 3 | 0 | 3 | 4 | - | 0 | 3 | 0 | 2 | 2 | 3 | 1 | 4 | 4 | 0 | 0 | - | 4 | 1 | - | 4 | 4 | 2 | 2 | 2 |
| 19 | 5 | Pre | 3 | 3 | 0 | 4 | 0 | 0 | 1 | 2 | 0 | 4 | 3 | 4 | 3 | - | 1 | - | - | 0 | 2 | 0 | - | 3 | 2 | 2 | 3 | 5 |
| | | Post | 3 | 3 | 4 | 2 | 0 | 0 | 0 | 1 | 1 | 3 | 2 | 3 | 1 | 0 | 3 | 0 | 0 | - | 1 | 0 | - | 2 | 0 | 0 | 0 | 1 |
| 20 | 5 | Pre | 3 | 4 | 3 | 3 | 4 | 5 | 4 | 4 | 4 | 0 | 3 | 4 | 5 | - | 4 | - | - | 5 | 5 | 5 | - | 5 | 5 | 4 | 3 | 3 |
| | | Post | 2 | 3 | 1 | 4 | 2 | 3 | 2 | 3 | 0 | 1 | 1 | 4 | 1 | 0 | 3 | 0 | 3 | - | 4 | 4 | - | 4 | 2 | 1 | 1 | 1 |
| 21 | 5 | Pre | 1 | 4 | 1 | 3 | 4 | 5 | 5 | 4 | 0 | 4 | 4 | 4 | 4 | 0 | 4 | - | - | 5 | 5 | 5 | - | 5 | 5 | 5 | 4 | 5 |
| | | Post | 1 | 4 | 2 | 3 | 4 | 4 | 1 | 3 | 1 | 4 | 2 | - | 3 | 4 | 4 | 3 | 2 | - | 4 | 3 | - | 5 | 4 | 2 | 1 | 2 |

Table IV (cont/d)

| COMPOUND NO | RATE OF APPLN kg/ha | PRE- OR POST-EMERGENCE APPLN. | TEST PLANTS (see Table VI) Sb | Rp | Ct | Sy | Mz | Ww | Rc | Sn | Ip | Am | Pi | Ca | Po | Xa | Ab | Cv | Av | Ot | Dg | Pu | Al | St | Ec | Sh | Ag | Cn |
|---|---|---|---|---|---|---|---|---|---|---|---|---|---|---|---|---|---|---|---|---|---|---|---|---|---|---|---|---|
| 22 | 5 | Pre | 4 | 4 | 2 | 4 | 1 | 0 | 0 | 4 | 1 | 4 | 4 | 4 | 4 | 4 | 4 | - | - | 2 | 4 | 5 | - | 5 | 5 | 3 | 5 | 5 |
| | | Post | 2 | 3 | 2 | 3 | 1 | 0 | 0 | 4 | 0 | 0 | 2 | 3 | 0 | 4 | 3 | 0 | 0 | - | 2 | 0 | - | 0 | 0 | 0 | 0 | 3 |
| 23 | 5 | Pre | 4 | 4 | 3 | 4 | 5 | 5 | 5 | 4 | 0 | 4 | - | 4 | 4 | 3 | 4 | - | - | 5 | 5 | 5 | - | 5 | 5 | 5 | 5 | 5 |
| | | Post | 2 | 4 | 4 | 4 | 2 | 1 | 3 | 3 | 2 | 4 | 4 | 4 | 4 | 3 | 3 | 2 | 2 | - | 4 | 4 | - | 4 | 4 | 4 | 3 | 3 |
| 24 | 5 | Pre | 4 | 4 | 3 | 4 | 4 | 5 | 4 | 4 | 0 | 4 | 4 | 4 | 4 | - | 4 | - | - | 5 | 5 | 5 | - | 5 | 5 | 4 | 5 | 5 |
| | | Post | 3 | 4 | 2 | 4 | 3 | 2 | 2 | 3 | 2 | 2 | 3 | 3 | 3 | 4 | 2 | 0 | 1 | - | 3 | 3 | - | 3 | 3 | 2 | 2 | 2 |
| 25 | 5 | Pre | 1 | 3 | 1 | 2 | 1 | 2 | 0 | 2 | 0 | 2 | 4 | 4 | 4 | 0 | 2 | - | - | 3 | 5 | 4 | - | 4 | 4 | - | 3 | 3 |
| | | Post | 3 | 3 | 3 | 4 | 0 | 0 | 3 | 1 | 0 | - | 3 | 4 | - | - | 3 | 0 | 0 | - | 1 | - | - | 4 | 3 | 0 | 0 | 2 |

Table IV (cont/d)

| COMPOUND NO | RATE OF APPLN kg/ha | PRE- OR POST-EMERGENCE APPLN. | TEST PLANTS (see Table VI) Sb | Rp | Ct | Sy | Mz | Ww | Rc | Sn | Ip | Am | Pi | Ca | Po | Xa | Ab | Cv | Av | Ot | Dg | Pu | Al | St | Ec | Sh | Ag | Cn |
|---|---|---|---|---|---|---|---|---|---|---|---|---|---|---|---|---|---|---|---|---|---|---|---|---|---|---|---|---|
| 26 | 5 | Pre | 0 | 3 | 0 | 4 | 4 | 2 | 1 | 4 | 0 | 3 | - | 3 | - | 0 | 3 | - | - | 1 | 4 | 5 | - | 5 | 4 | 4 | 5 | 4 |
| | | Post | 1 | 3 | 3 | 4 | 1 | 1 | 1 | 2 | 2 | 2 | 2 | 4 | 2 | 0 | 3 | 0 | 0 | - | 4 | 2 | - | 2 | 2 | 2 | 0 | 2 |
| 33 | 5 | Pre | 4 | 4 | 2 | 4 | 0 | 1 | 0 | 4 | 0 | 4 | 4 | - | 4 | 2 | 4 | - | - | 1 | 3 | 3 | - | 4 | 3 | 0 | - | 1 |
| | | Post | 0 | 3 | 2 | 4 | 0 | 0 | 0 | 1 | 0 | 3 | 0 | 4 | 1 | 2 | 3 | 0 | 1 | - | 3 | 1 | - | 2 | 0 | 0 | 0 | 0 |
| 34 | 1 | Pre | 3 | 4 | 2 | 3 | 2 | 2 | 0 | 4 | 0 | 4 | 4 | 4 | 4 | 1 | 1 | - | - | 0 | 4 | - | - | 4 | 3 | 1 | 4 | 3 |
| | | Post | 1 | 3 | 0 | 3 | 0 | 1 | 0 | 3 | 0 | 1 | 2 | 1 | 1 | 4 | 2 | 0 | 0 | 0 | 0 | 0 | - | 0 | 0 | 0 | 0 | 0 |
| 38 | 5 | Pre | 1 | 3 | 0 | 4 | 0 | 0 | 0 | 1 | 0 | 3 | - | 4 | 2 | 0 | 2 | - | - | 0 | 0 | 3 | - | 2 | 1 | 0 | 0 | 1 |
| | | Post | 0 | 2 | 0 | 1 | 0 | 0 | 0 | 2 | 2 | 0 | 1 | 3 | 0 | 0 | 1 | 0 | 0 | - | 0 | 0 | - | 1 | 0 | 0 | 0 | 0 |

Table IV (cont/d)

| COMPOUND NO | RATE OF APPLN kg/ha | PRE- OR POST-EMERGENCE APPLN. | TEST PLANTS (see Table VI) | | | | | | | | | | | | | | | | | | | | | | | | | |
|---|---|---|---|---|---|---|---|---|---|---|---|---|---|---|---|---|---|---|---|---|---|---|---|---|---|---|---|---|
| | | | Sb | Rp | Ct | Sy | Mz | Ww | Rc | Sn | Ip | Am | Pi | Ca | Po | Xa | Ab | Cv | Av | Ot | Dg | Pu | Al | St | Ec | Sh | Ag | Cn |
| 39 | 5 | Pre | 2 | 3 | 1 | 0 | 1 | 2 | 0 | 4 | 0 | 2 | - | 4 | 3 | 0 | 2 | - | - | 0 | 3 | 4 | - | 4 | 3 | 1 | 2 | 0 |
| | | Post | 2 | 3 | 0 | 0 | 0 | 0 | 0 | 2 | 0 | 1 | 2 | 3 | 0 | 0 | 1 | 0 | 0 | - | 0 | 1 | - | 1 | 0 | 0 | 0 | 2 |
| 41 | 5 | Pre | 2 | 1 | 3 | 0 | 0 | 0 | 0 | 3 | 0 | 2 | 2 | 3 | 0 | - | 2 | - | - | 0 | 3 | 1 | - | 1 | 1 | 0 | 0 | 0 |
| | | Post | 1 | 3 | 2 | 2 | 1 | 0 | 0 | 0 | 0 | 2 | 0 | 2 | 2 | 0 | 0 | 0 | 0 | - | 0 | 0 | - | 0 | 1 | 0 | 0 | 0 |
| 140 | 5 | Pre | 3 | 4 | 3 | 2 | 3 | 1 | 2 | 4 | 1 | 3 | 3 | 4 | 3 | - | 3 | - | - | 4 | 4 | 5 | - | 4 | 5 | 3 | 1 | 1 |
| | | Post | 2 | 4 | 0 | 3 | 2 | 0 | 0 | 1 | 2 | 2 | 1 | 3 | 2 | 0 | - | 3 | 0 | 0 | 2 | 2 | - | 3 | 3 | 0 | 0 | 2 |
| 141 | 5 | Pre | 0 | 2 | 0 | 0 | 2 | 0 | 1 | 2 | 0 | 0 | 1 | 2 | 2 | - | 2 | - | - | 0 | 3 | 5 | - | 3 | 4 | 3 | 2 | 1 |
| | | Post | 0 | 0 | 0 | 0 | 0 | 0 | 0 | 0 | 0 | 0 | 0 | 0 | 0 | 0 | 0 | 0 | 1 | - | 0 | 0 | - | 1 | - | 1 | 0 | 0 |

TABLE IV (cont/d)

| COMPOUND NO | RATE OF APPLN kg/ha | PRE- OR POST-EMERGENCE APPLN. | Sb | Rp | Ct | Sy | Mz | Ww | Rc | Sn | Ip | Am | Pi | Ca | Po | Xa | Ab | Cv | Av | Ot | Dg | Pu | Al | St | Ec | Sh | Ag | Cn |
|---|---|---|---|---|---|---|---|---|---|---|---|---|---|---|---|---|---|---|---|---|---|---|---|---|---|---|---|---|
| | | | TEST PLANTS (see Table VI) | | | | | | | | | | | | | | | | | | | | | | | | | |
| 142 | 5 | Pre | 3 | 4 | 0 | 2 | 0 | 2 | 3 | 4 | 0 | 2 | 2 | 3 | 2 | - | 3 | - | 4 | 5 | 5 | - | 4 | 5 | 0 | 0 | 0 | 0 |
| | | Post | 2 | 1 | 0 | 2 | 1 | 0 | 0 | 2 | 1 | 1 | 1 | 3 | 2 | 0 | 2 | 0 | 1 | - | 3 | 1 | - | 4 | 1 | 1 | 2 | 0 |
| 143 | 5 | Pre | 2 | 4 | 1 | 3 | 5 | 2 | 2 | 4 | 0 | 2 | 3 | 4 | 4 | 0 | 4 | - | - | 4 | 5 | 5 | - | 5 | 4 | 0 | 0 | 0 |
| | | Post | 1 | 2 | 4 | 3 | 1 | 3 | 0 | 2 | 1 | 1 | 1 | 3 | 2 | 0 | 3 | 0 | 0 | - | 4 | 0 | - | 2 | 0 | 2 | 0 | 2 |
| 145 | 5 | Pre | 2 | 3 | 1 | 4 | 3 | 2 | 2 | 3 | 0 | 3 | - | 4 | 3 | 0 | 3 | - | - | 1 | 3 | 5 | - | 4 | 3 | 1 | 2 | 0 |
| | | | 1 | 3 | 2 | 1 | 0 | 0 | 0 | 2 | 2 | 2 | 0 | 4 | 2 | 0 | 2 | 0 | 0 | - | 0 | 0 | - | 3 | 2 | 0 | 0 | 0 |

TABLE V

| COMPOUND NO | RATE OF APPLN kg/ha | PRE- OR POST-EMERGENCE APPLN. | TEST PLANTS (see Table VI) | | | | | | | | | | | | | | | | | | | | | | | | |
|---|---|---|---|---|---|---|---|---|---|---|---|---|---|---|---|---|---|---|---|---|---|---|---|---|---|---|---|
| | | | Sb | Rp | Ct | Sy | Mz | Rc | Ww | Pi | Ca | Ga | Am | Bd | Eh | Ip | Ab | Xa | Xs | Av | Al | Ag | Sh | St | Dg | Ec | Ce |
| 71 | 4 | Pre | 0 | 75 | 0 | 0 | 10 | 0 | 0 | - | - | - | 30 | 50 | 0 | 3 | 85 | 0 | - | 0 | 95 | - | 85 | 95 | - | 95 | 50 |
| | 4 | Post | 10 | 3 | 3 | 50 | 0 | 0 | 0 | - | 50 | - | 50 | 65 | 0 | 0 | 50 | - | 0 | 0 | 0 | 0 | 10 | 10 | 10 | 20 | - |
| 74 | 1 | Pre | 0 | 0 | 50 | 5 | 0 | - | - | 35 | 0 | 0 | 0 | 0 | 0 | 10 | 60 | 0 | - | 0 | 0 | - | 97 | 97 | - | 97 | 65 |
| | 4 | Post | 0 | 70 | 50 | 35 | 70 | 0 | 0 | 0 | 50 | 70 | 45 | 70 | 25 | 40 | 35 | - | 30 | 50 | 65 | 20 | 45 | 70 | 60 | 75 | 40 |
| 75 | 1 | Pre | 10 | 20 | 10 | 0 | 0 | 10 | 0 | - | 75 | - | 0 | 20 | 20 | 0 | 75 | 10 | - | 20 | 95 | - | 95 | 95 | - | 95 | 50 |
| | 1 | Post | 50 | 30 | 30 | 65 | 50 | 0 | 0 | 0 | 50 | 0 | 0 | 50 | 30 | 0 | 50 | - | 20 | 0 | 0 | 10 | 20 | 50 | 65 | 20 | 0 |
| 78 | 4 | Pre | 0 | 85 | 3 | 0 | - | 0 | 0 | 30 | 50 | 20 | 10 | 30 | 30 | 0 | 85 | 0 | - | 0 | 0 | - | 85 | 95 | 95 | - | 95 |
| | 4 | Post | 30 | 50 | 65 | 75 | 50 | 0 | 0 | 0 | 75 | 0 | 75 | 50 | 20 | 30 | 75 | - | 30 | 0 | 10 | 0 | 20 | 30 | 20 | 0 | 0 |

TABLE V

| COMPOUND NO | RATE OF APPLN kg/ha | PRE- OR POST-EMERGENCE APPLN. | TEST PLANTS (see Table VI) | | | | | | | | | | | | | | | | | | | | | | | |
|---|---|---|---|---|---|---|---|---|---|---|---|---|---|---|---|---|---|---|---|---|---|---|---|---|---|---|---|
| | | | Sb | Rp | Ct | Sy | Mz | Rc | Ww | Pi | Ca | Ga | Am | Bd | Eh | Ip | Ab | Xa | Xs | Av | Al | Ag | Sh | St | Dg | Ec | Ce |
| 79 | 1 | Pre | 0 | 30 | 30 | 0 | 0 | 0 | 0 | 95 | 50 | - | 50 | 50 | 50 | 20 | 50 | 20 | - | 30 | 85 | - | 50 | 95 | - | 95 | 50 |
| | 1 | Post | 10 | 50 | 0 | 10 | 20 | 0 | 0 | - | 85 | - | 0 | 10 | 30 | 0 | 30 | - | 0 | 0 | 0 | 0 | 0 | 0 | 0 | 0 | - |
| 81 | 4 | Pre | 50 | 95 | 0 | 50 | 0 | 65 | 0 | 0 | 20 | - | 75 | 75 | 75 | - | 50 | 0 | - | 30 | 65 | - | 30 | 95 | - | 95 | 0 |
| | 4 | Post | 50 | - | 20 | 50 | 0 | 0 | 0 | - | 85 | - | 20 | 30 | 20 | 30 | 30 | - | 0 | 10 | 0 | 0 | 10 | 95 | 3 | 10 | 0 |
| 83 | 1 | Pre | 0 | 30 | 0 | 0 | 3 | 85 | 0 | - | 75 | - | 10 | 20 | 20 | - | 30 | 0 | 0 | 30 | 95 | - | 85 | 95 | - | 95 | 50 |
| | 4 | Post | 65 | 50 | 65 | 65 | 95 | 30 | 65 | 30 | 85 | 20 | 65 | 95 | 50 | 30 | 65 | - | 65 | 50 | 50 | 50 | 50 | 95 | 95 | 95 | 50 |
| 88 | 1 | Pre | 0 | 5 | 80 | 0 | 0 | 0 | 0 | 35 | 50 | 5 | 0 | 0 | 0 | 0 | 75 | 0 | - | 5 | 35 | - | 95 | 100 | - | 100 | 20 |
| | 4 | Post | 45 | 70 | 40 | 55 | 60 | 0 | - | 30 | 70 | 10 | 45 | 45 | 65 | 10 | 55 | - | 25 | 20 | 40 | 20 | 60 | 70 | 75 | 45 | 40 |

TABLE V

| COMPOUND NO | RATE OF APPLN kg/ha | PRE- OR POST-EMERGENCE APPLN. | TEST PLANTS (see Table VI) | | | | | | | | | | | | | | | | | | | | | | | |
|---|---|---|---|---|---|---|---|---|---|---|---|---|---|---|---|---|---|---|---|---|---|---|---|---|---|---|
| | | | Sb | Rp | Ct | Sy | Mz | Rc | Ww | Pi | Ca | Ga | Am | Bd | Eh | Ip | Ab | Xa | Xs | Av | Al | Ag | Sh | St | Dg | Ec | Ce |
| 91 | 4 | Pre | 0 | 20 | 0 | 0 | 30 | 0 | 3 | 0 | 65 | - | 0 | 0 | 0 | 0 | 20 | 0 | - | 0 | 0 | - | 30 | 95 | 95 | 95 | 0 |
| | 4 | Post | 10 | 50 | 65 | 10 | 3 | 0 | 0 | 10 | 0 | - | 0 | 30 | 65 | 0 | 75 | - | 10 | 95 | 75 | 0 | 50 | 0 | 0 | 0 | 0 |
| 116 | 1 | Pre | 10 | 20 | 0 | 0 | 20 | 10 | 0 | - | 50 | - | 50 | 20 | 20 | 0 | 20 | 0 | - | 50 | 75 | - | 65 | 95 | - | 95 | 30 |
| | 1 | Post | 50 | 20 | 65 | 65 | 50 | 3 | 0 | 0 | 10 | 0 | 0 | 50 | 50 | 30 | 30 | - | 30 | 10 | 3 | 0 | 30 | 3 | 10 | 20 | 0 |

TABLE VI

| | |
|---|---|
| Sb | Sugar beet |
| Rp | Rape |
| Ct | Cotton |
| Sy | Soya bean |
| Mz | Maize |
| Ww | Winter wheat |
| Rc | Rice |
| Sn | Senecio vulgaris |
| Ip | Ipomoea purpurea |
| Am | Amaranthus retroflexus |
| Pi | Polygonum aviculare |
| Ca | Chenopodium album |
| Po | Portulaca oleracea |
| Xa | Xanthium spinosum |
| Ab | Abutilon theophrastii |
| Cv | Convolvulus arvensis |
| Ot/Av | Oats (cultivated in pre-emergence test and Avena fatua (wild oats) in post-emergence test) |
| Dg | Digitaria sangiunalis |
| Pu | Poa annua |
| Al | Alopecuris myosuroides |
| St | Setaria viridis |
| Ec | Echinochloa crus-galli |
| Sh | Sorghum halepense |
| Ag | Agropyron repens |
| Cn | Cyperus rotundus |
| Ga | Galium aparine |
| Am | Amaranthus retroflexus |
| Bd | Bidens pilosa |
| Eh | Euphorbia heterophylla |
| Xs | Xanthium strumarium |
| Ce | Cyperus esculentes |

CHEMICAL FORMULAE

(in description)

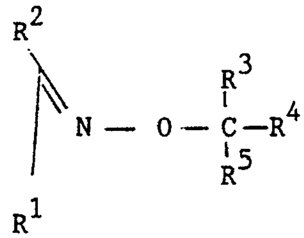

(I)

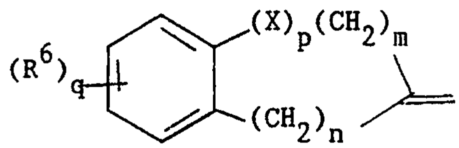

(i)

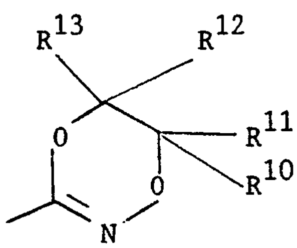

(ii)

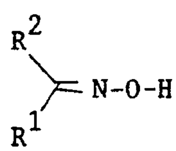

(II)

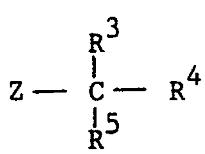

(III)

$H\text{-}NR^8R^9$ (VII)

$AlR^{16}R^{17}R^{18}$ (VI)

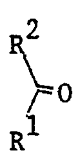

(IV)

$R^9R^8N - Al\ R^{16}R^{17}$ (V)

## Claims

1. A compound of formula (I):

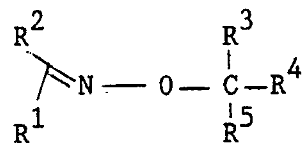

(I)

where $R^1$ is alkyl, optionally substituted aryl or optionally substituted heterocyclyl;
$R^2$ is hydrogen, optionally substituted lower alkyl, lower alkoxy, cycloalkyl, haloalkyl, lower thioalkyl, halo or cyano; or the group $R^1$ is a group of sun formula (i):

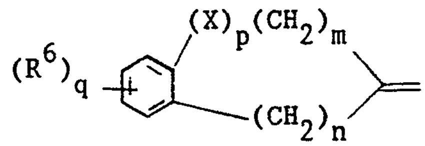

(i)

where q is 0 or an integer of from 1 to 4 and groups $R^6$ are selected independently from hydroxy, alkyl, alkoxy, alkylcarbonyl, halogen, nitrile, nitro, haloalkyl and haloalkoxy; X is oxygen or sulphur, p is 0 or 1, n is 0 or 1 and m is 1, 2 or 3 provided that when n is 0, p+ m is 2 or 3 and when n is 1, p+ m is 1 or 2;
$R^3$ and $R^4$ are independently selected from hydrogen, halo, alkyl or alkoxy or $R^3$ and $R^4$ together with the carbon atom to which they are attached form a carbocyclic ring; and
$R^5$ is CN; $CO_2R^7$ where $R^7$ is hydrogen, a cation or an esterifying group; a group $-CYNR^8R^9$ where Y is oxygen or sulphur and $R^8$ and $R^9$ are independently selected from hydrogen, hydroxy, alkyl or alkoxy or together with the nitrogen atom to which they are attached form a heterocyclic ring; or $R^8$ together with $R^3$ and the group

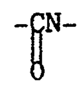

to which it is attached form a heterocyclic ring, or $R^5$ is a group of sub formula (ii)

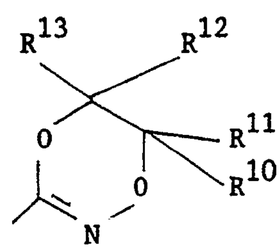

(ii)

where $R^{10}$, $R^{11}$, $R^{12}$ and $R^{13}$ are independently selected from hydrogen or alkyl; or a group S(O)r $R^{14}$ where r is 0, 1 or 2 and $R^{14}$ is hydroxy, lower alkyl or aryl.

**2.** A compound according to claim 1 where $R^3$ and $R^4$ are other than hydrogen.

**3.** A compound according to claim 1 or claim 2 where $R^5$ is a group $CYNR^8R^9$.

**4.** A compound according to claim 3 where Y is oxygen.

**5.** A compound according to any one of claims 1 to 4 where $R^1$ is optionally substituted phenyl.

**6.** A compound according to claim 5 wherein the phenyl ring $R^1$ has at least one substitutuent at the ortho position on the ring.

**7.** A herbicidal composition comprising a compound of formula (I) as defined in claim 1 in combination with an agriculturally acceptable carrier or diluent.

**8.** A composition according to claim 7 which further comprises another herbicidal compound.

**9.** A method of killing or controlling unwanted plants which methyl comprises applying to the plants or to the locus thereof an effective amount of a compound of formula (I) as defined in claim 1.

**10.** A process for preparing a compound of formula (I) as defined in claim 1 which process comprises reacting a compound of formula (II):

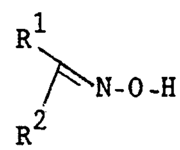

(II)

wherein $R^1$ and $R^2$ are as defined in relation to formula (I); with a compound of formula (III):

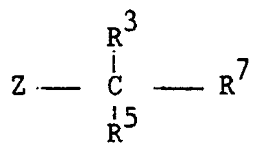

(III)

wherein $R^3$, $R^4$ and $R^5$ are as defined in relation to formula (I) and Z is a leaving group in the presence of a base; and thereafter if desired coverting the group $R^5$ to a different such group.

European Patent Office

# EUROPEAN SEARCH REPORT

Application Number

EP 91305039.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US - A - 4 548 756 (HENRY MARTIN) * Abstract; claims 1-5; column 11, lines 38-51 * -- | 1,5, 6-10 | C 07 C 251/60 C 07 C 323/12 A 01 N 37/36 |
| X | EP - A1 - 0 182 407 (SHELL INTERNATIONALE) * Claims 1,2,3,5,6 * -- | 1,2,5, 6,7,9 | |
| X | CH - A5 - 657 358 (CIBA-GEIGY) * Claims 1,6-8 * -- | 1,5, 7-9 | |
| X | EP - A1 - 0 077 854 (UNION CARBIDE) * Claims 1-8,16 * -- | 1,3-7, 9 | |
| X | CH - A - 616 406 (SIEGFRIED AKTIENGESELLSCHAFT) * Abstract * -- | 1-5,7, 10 | TECHNICAL FIELDS SEARCHED (Int. Cl.5) |
| X | DE - A - 1 960 910 (N.V. PHILIPS'GLOEILAMPEN-FABRIEKEN) * Claims 1,2,11 * -- | 1-6,10 | C 07 C 251/00 C 07 C 317/00 C 07 C 323/00 C 07 C 327/00 |
| A | DE - A1 - 3 017 795 (PPG INDUSTRIES) * Claims 1,17 * ---- | 1,7,9 | |

The present search report has been drawn up for all claims

| Place of search | Date of completion of the search | Examiner |
|---|---|---|
| VIENNA | 03-09-1991 | REIF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)